# EUROPEAN PATENT SPECIFICATION

(11) **EP 3 284 442 B1**
(45) Date of publication and mention of the grant of the patent: **19.07.2023**
(21) Application number: 17183637.2
(22) Date of filing: 27.07.2017
(51) Int. Cl.: A61F 2/40, A61B 17/17

(54) **STABILIZED DRILL GUIDE**
STABILISIERTE BOHRFÜHRUNG
GUIDE-FORET STABILISÉ

(30) Priority: 27.07.2016 US 201662367533 P; 04.08.2016 US 201615228443; 18.07.2017 US 201715653305; 26.07.2017 US 201715660942
(43) Date of publication of application: 21.02.2018
(73) Proprietor: Catalyst OrthoScience Inc., Naples, FL 34110 (US)
(72) Inventor: GOLDBERG, Steven S., Naples, Florida 34110 (US)
(74) Representative: HGF

(56) References cited:
- WO-A1-2011/029911
- US-A- 5 851 207
- US-A1- 2003 134 252
- US-A1- 2005 038 444
- US-A1- 2014 163 565
- US-B1- 8 986 309

## Description

### TECHNICAL FIELD

The present disclosure relates to a drill guide for use in surgery. More specifically, the present disclosure relates to a drill guide for orthopedic surgery, such as glenoid arthroplasty. One of skill in the art will appreciate that the principles set forth herein are applicable to other types of surgery where a drill guide is used.

### BACKGROUND

Orthopedic implants are designed to be rigidly fixed within bone, both with and without the use of bone cement. It is imperative than the implants are seated within bone in channels that precisely match the actual shape of the implants in order for them to lie flat or otherwise seat in the way to minimize chance of failure. If the channels created for the implant to seat are in an improper position, orientation, size, or incorrect depth, the implant may not seat correctly, and may be prone to abnormal forces, loosening, or may not function properly.

Loosening of an implanted orthopedic device can cause pain, loss of motion and further tissue destruction, possibly leading to the implant needing to be removed or revised. Reoperation for loose orthopedic implants is a major source of increased costs to the healthcare system. Therefore, it is important that implants are implanted precisely the way they were engineered to be placed in order maximize the chance of success. Documents US2014163565 A1, US5851207 A and US2005038444 A1 disclose exemplary drill guides.

### SUMMARY

The various systems of the present technology have been developed in response to the present state of the art, and in particular, in response to the problems and needs in the art that have not yet been fully solved by currently available drill guides. The systems of the present technology may provide meaningful improvement in the stability of the drill guide working portion, thereby improving the accuracy of the prepared bone socket, thereby improving the implanted position of the corresponding prosthesis (to be closer to the nominal design implant position).

To achieve the foregoing, and in accordance with the technology as embodied and broadly described herein, an aspect of the technology includes a drill guide including: a working portion including a bone-facing side, an opposite obverse side, and a through hole that extends through at least a portion of the working portion, wherein the through hole receives a drill with clearance; and a shaft coupled to the working portion by a joint, wherein the shaft is movable relative to the working portion about the joint.

Embodiments of this aspect may include one or more of the following attributes. The joint is selected from the group consisting of a hinge joint, a universal joint, a ball-and-socket joint, a polyaxial joint, a saddle joint, a flexible shaft portion, and a magnetic joint. The joint is a hinge joint between the shaft and the working portion. The working portion is captive to the shaft. The shaft is movable relative to the working portion about the joint while the drill is actuated in the through hole. The shaft is movable relative to the working portion about the joint within a range of motion, wherein the obverse side includes at least one range of motion stop associated with the joint. The drill guide includes a biasing element that biases the shaft to contact the at least one range of motion stop. The biasing element is selected from the group consisting of a spring, a magnet, a cam, and a drag pin. The at least one range of motion stop includes a ridge on the obverse side, wherein the shaft contacts the ridge at a first end of the range of motion. The drill guide includes a locking mechanism including a first setting and a second setting; wherein when the locking mechanism is in the first setting, the shaft is immobilized relative to the working portion; wherein when the locking mechanism is in the second setting, the shaft is freely movable relative to the working portion. The bone-facing side includes a stabilizing feature that stabilizes the working portion relative to a bone. The stabilizing feature includes a spike protruding from the bone-facing side. The working portion includes at least one anatomical reference feature. The working portion includes a pair of outwardly extending anatomical reference tabs on opposite sides of the working portion. When the bone-facing side is placed against a glenoid socket, the working portion is positionable so that the pair of outwardly extending anatomical reference tabs are aligned along a superior-inferior direction.

Another aspect of the technology includes a drill guide including: a working portion including a through hole, wherein the through hole receives a drill with clearance; and a handle portion coupled to the working portion by a joint, wherein the handle portion is movable relative to the working portion about the joint.

Embodiments of this aspect may include one or more of the following attributes. The joint is selected from the group consisting of a hinge joint, a universal joint, a ball-and-socket joint, a polyaxial joint, a saddle joint, a flexible shaft portion, and a magnetic joint. The joint is a hinge joint between the handle portion and the working portion. The working portion is captive to the handle portion. The handle portion is movable relative to the working portion about the joint while the drill is actuated in the through hole. The handle portion is movable relative to the working portion about the joint within a range of motion, wherein the obverse side includes at least one range of motion stop associated with the joint. The drill guide includes a biasing element that biases the handle portion to contact the at least one range of motion stop. The biasing element is selected from the group consisting of a spring, a magnet, a cam, and a drag pin. The at least one range of motion stop includes a ridge on the obverse side, wherein the handle portion contacts the ridge at a first end of the range of motion. The drill guide includes a locking mechanism including a first setting and a second setting; wherein when the locking mechanism is in the first setting, the handle portion is immobilized relative to the working portion; wherein when the locking mechanism is in the second setting, the handle portion is freely movable relative to the working portion. The working portion includes a bone-facing side includes a stabilizing feature that stabilizes the working portion relative to a bone. The stabilizing feature includes a spike protruding from the bone-facing side. The working portion includes at least one anatomical reference feature. The working portion includes a pair of outwardly extending anatomical reference tabs on opposite sides of the working portion. When the bone-facing side is placed against a glenoid socket, the working portion is positionable so that the pair of outwardly extending anatomical reference tabs are aligned along a superior-inferior direction. The through hole may extend through at least a portion of the working portion.

Yet another aspect of the technology includes a drill guide including: a working portion including a through hole and a first joint portion, wherein the through hole receives a drill with clearance; and a handle portion including a second joint portion, wherein the handle portion is coupled to the working portion by a joint formed by the first and second joint portions, wherein the handle portion is movable relative to the working portion about the joint.

Embodiments of this aspect may include one or more of the following attributes. The joint is selected from the group consisting of a hinge joint, a universal joint, a ball-and-socket joint, a polyaxial joint, a saddle joint, a flexible shaft portion, and a magnetic joint. The joint is a hinge joint between the handle portion and the working portion. The working portion is captive to the handle portion. The handle portion is movable relative to the working portion about the joint while the drill is actuated in the through hole. The handle portion is movable relative to the working portion about the joint within a range of motion, wherein the obverse side includes at least one range of motion stop associated with the joint. The drill guide includes a biasing element that biases the handle portion to contact the at least one range of motion stop. The biasing element is selected from the group consisting of a spring, a magnet, a cam, and a drag pin. The at least one range of motion stop includes a ridge on the obverse side, wherein the handle portion contacts the ridge at a first end of the range of motion. The drill guide includes a locking mechanism including a first setting and a second setting; wherein when the locking mechanism is in the first setting, the handle portion is immobilized relative to the working portion; wherein when the locking mechanism is in the second setting, the handle portion is freely movable relative to the working portion. The working portion includes a bone-facing side includes a stabilizing feature that stabilizes the working portion relative to a bone. The stabilizing feature includes a spike protruding from the bone-facing side. The working portion includes at least one anatomical reference feature. The working portion includes a pair of outwardly extending anatomical reference tabs on opposite sides of the working portion. When the bone-facing side is placed against a glenoid socket, the working portion is positionable so that the pair of outwardly extending anatomical reference tabs are aligned along a superior-inferior direction. The through hole may extend through at least a portion of the working portion.

These and other features and advantages of the present technology will become more fully apparent from the following description and appended claims, or may be learned by the practice of the technology as set forth hereinafter.

### BRIEF DESCRIPTION OF THE DRAWINGS

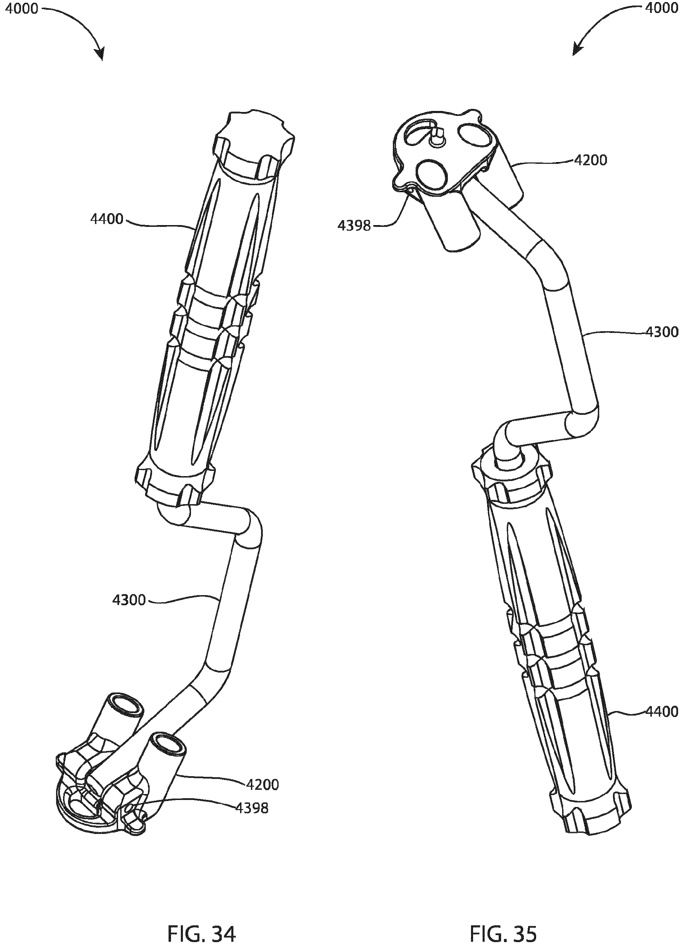

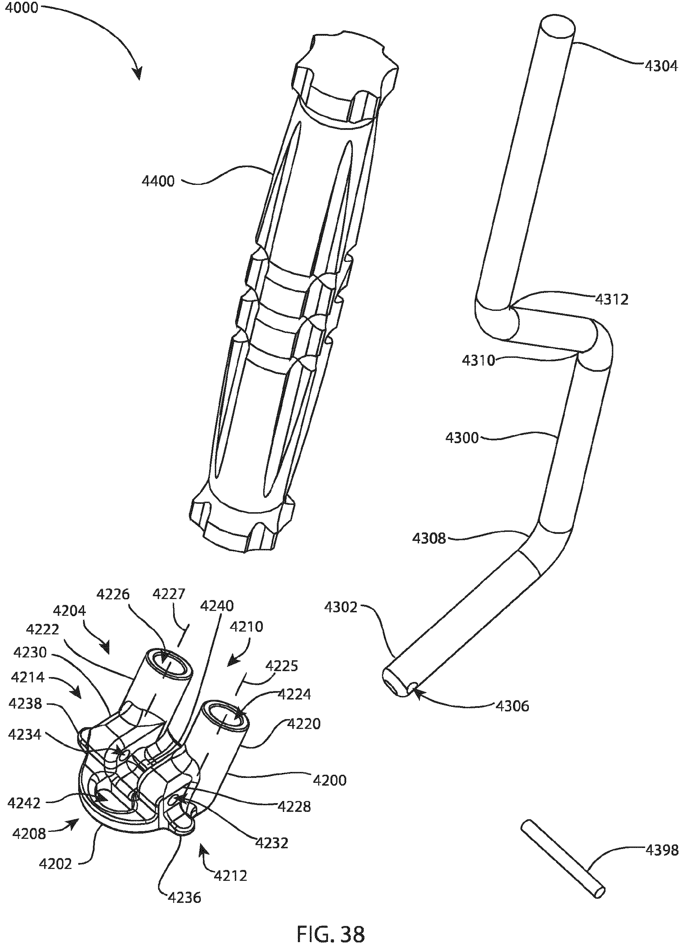

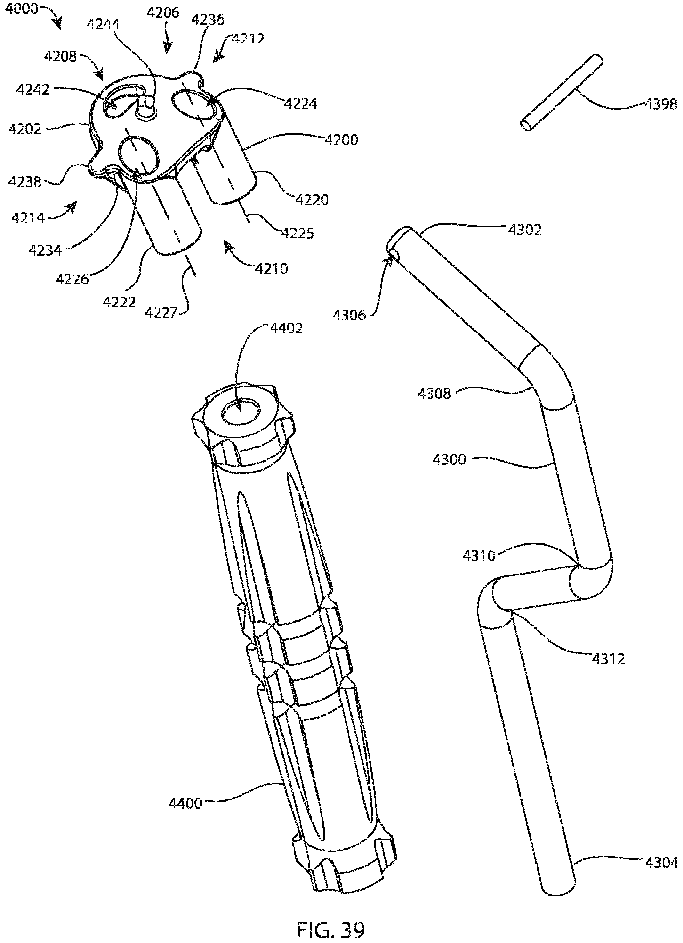

Exemplary embodiments of the technology will become more fully apparent from the following description and appended claims, taken in conjunction with the accompanying drawings. Understanding that these drawings depict only exemplary embodiments and are, therefore, not to be considered limiting of the scope of the technology, the exemplary embodiments will be described with additional specificity and detail through use of the accompanying drawings . Only figures 34 to 41 show the drill guide of the invention as claimed. In the accompanying drawings:
FIG. 1A is a superior-posterior-medial view of a glenoid component; and FIG. 1B is a superior-anterior-medial view of the glenoid component of FIG. 1A;
FIG. 2A is a superior-posterior-medial view of another glenoid component; and FIG. 2B is a superior-anterior-medial view of the glenoid component of FIG. 2A;
FIG. 3A is a superior-posterior-medial view of yet another glenoid component; and FIG. 3B is a superior-anterior-medial view of the glenoid component of FIG. 3A;
FIG. 4 is a superior-posterior-medial view of yet another glenoid component;
FIG. 5 is a superior-posterior-medial view of yet another glenoid component;
FIG. 6 is a superior-posterior-medial view of yet another glenoid component;
FIG. 7A is an inferior-anterior-medial view of yet another glenoid component; and FIG. 7B is a superior-anterior-lateral view of the glenoid component of FIG. 7A;
FIG. 8A is an isometric view of a size template; and FIG. 8B is a detail view of a working portion of the size template of FIG. 8A;
FIG. 9A is an isometric view of a reamer; and FIG. 9B is another isometric view of the reamer of FIG. 9A from a different direction;
FIG. 10A is an isometric view of a drill guide; and FIG. 10B is another isometric view of the drill guide of FIG. 22A from a different direction;
FIG. 11A is an isometric view of another drill guide with a drill and a keel position tamp; and FIG. 11B is another isometric view of the drill guide, drill, and keel position tamp of FIG. 11A from a different direction;
FIG. 12A is an isometric view of yet another drill guide; and FIG. 12B is an enlarged detail view of a portion of the drill guide of FIG. 12A;
FIG. 13A is an isometric view of yet another drill guide with drills; and FIG. 13B is another isometric view of the drill guide and drills of FIG. 13A from a different direction;
FIG. 14A is an isometric view of yet another drill guide with a drill; and FIG. 14B is another isometric view of the drill guide and drills of FIG. 14A from a different direction;
FIG. 15A is an isometric view of a punch; and FIG. 15B is another isometric view of the punch of FIG. 15A from a different direction;
FIG. 16A is an isometric view of a broach; FIG. 16B is a detail view of a working portion of the broach of FIG. 16A; and FIG. 16C is another detail view of a working portion of the broach of FIG. 16A from a different direction;
FIG. 17A is an isometric view of an offset reamer; and FIG. 17B is an isometric view of a portion of the offset reamer of FIG. 17A from a second viewpoint;
FIG. 18A is an isometric view of another offset reamer; and FIG. 18B is an isometric view of a portion of the offset reamer of FIG. 18A from a second viewpoint;
FIG. 19 is an isometric view of yet another offset reamer;
FIG. 20 is an isometric view of yet another offset reamer;
FIG. 21 is an isometric view of a left glenoid component;
FIG. 22 is an isometric view of another left glenoid component;
FIG. 23 is an isometric view of yet another left glenoid component;
FIG. 24 is an isometric view of yet another left glenoid component;
FIG. 25 is an isometric view of yet another left glenoid component;
FIG. 26 is an isometric view of yet another left glenoid component;
FIG. 27 is an isometric view of yet another left glenoid component;
FIG. 28A is a medial-superior-posterior view of yet another glenoid component; and FIG. 28B is a medial-superior-anterior view of the glenoid component of FIG. 28A;
FIG. 29A is a medial-superior-posterior view of yet another glenoid component; and FIG. 29B is a medial-superior-anterior view of the glenoid component of FIG. 29A;
FIG. 30A is a medial-superior-posterior view of yet another glenoid component; and FIG. 30B is a medial-superior-anterior view of the glenoid component of FIG. 30A;
FIG. 31A is a medial-superior-posterior view of yet another glenoid component; and FIG. 31B is a medial-superior-anterior view of the glenoid component of FIG. 31A;
FIG. 32A is a medial-superior-posterior view of yet another glenoid component; and FIG. 32B is a medial-superior-anterior view of the glenoid component of FIG. 32A;

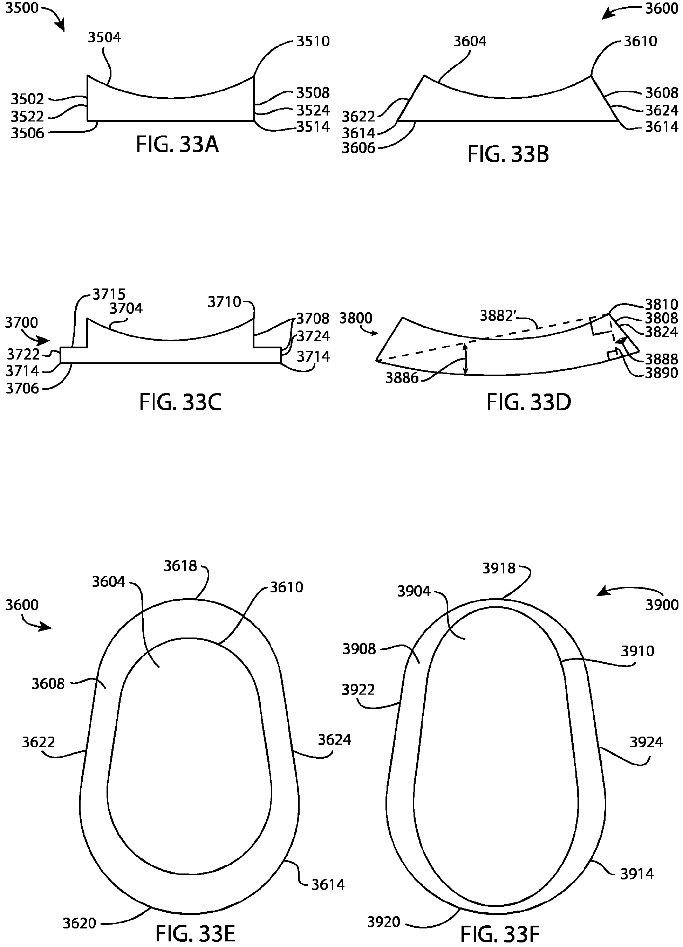

FIG. 33A is a cross section of yet another glenoid component, taken across the anterior-poster width of the glenoid component; FIG. 33B is a cross section of yet another glenoid component, taken across the anterior-poster width of the glenoid component; FIG. 33C is a cross section of yet another glenoid component, taken across the anterior-poster width of the glenoid component; FIG. 33D is a cross section of yet another glenoid component, taken across the anterior-posterior width of the glenoid component; FIG. 33E is a lateral view of the glenoid component of FIG. 33B; and FIG. 33F is a lateral view of yet another glenoid component;
FIG. 34 is an isometric view of a drill guide;
FIG. 35 is another isometric view of the drill guide of FIG. 34 from a different direction;
FIG. 36A is a front view of the drill guide of FIG. 34;
FIG. 36B is a cross sectional detail view of a portion of the drill guide of FIG. 34, taken along section line 36B-36B of FIG. 3A;
FIG. 37 is a side view of the drill guide of FIG. 34;
FIG. 38 is an isometric exploded view of the drill guide of FIG. 34;
FIG. 39 is another isometric exploded view of the drill guide of FIG. 34 from a different direction;
FIG. 40 is an isometric view of a working portion of the drill guide of FIG. 34;
FIG. 41 is another isometric view of the working portion of FIG. 40 from a different direction;
FIG. 42 is a superior view of the glenoid component of FIG. 26 implanted in a prepared bone socket in a glenoid fossa of a scapula; and
FIG. 43 is a superior view of the glenoid component of FIG. 26 implanted in another prepared bone socket in a glenoid fossa of a scapula.

### DETAILED DESCRIPTION

Exemplary embodiments of the technology will be best understood by reference to the drawings, wherein like parts are designated by like numerals throughout. It will be readily understood that the components of the technology, as generally described and illustrated in the figures herein, could be arranged and designed in a wide variety of different configurations. Thus, the following more detailed description of the embodiments of the apparatus is not intended to limit the scope of the invention, as claimed, but is merely representative of exemplary embodiments of the technology.

The phrases "connected to," "coupled to" and "in communication with" refer to any form of interaction between two or more entities, including mechanical, electrical, magnetic, electromagnetic, fluid, and thermal interaction. Two components may be functionally coupled to each other even though they are not in direct contact with each other. The term "abutting" refers to items that are in direct physical contact with each other, although the items may not necessarily be attached together. The phrase "fluid communication" refers to two features that are connected such that a fluid within one feature is able to pass into the other feature.

The word "exemplary" is used herein to mean "serving as an example, instance, or illustration." Any embodiment described herein as "exemplary" is not necessarily to be construed as preferred or advantageous over other embodiments. While the various aspects of the embodiments are presented in drawings, the drawings are not necessarily drawn to scale unless specifically indicated.

Standard medical planes of reference and descriptive terminology are employed in this specification. A sagittal plane divides a body into right and left portions. A mid-sagittal plane divides the body into bilaterally symmetric right and left halves. A coronal plane divides a body into anterior and posterior portions. A transverse plane divides a body into superior and inferior portions. The sagittal, coronal, and transverse planes are mutually perpendicular. A scapular plane is parallel to the body of the scapula and normal to the glenoid articular surface; the scapular plane is normally 30 to 45 degrees anterior of the coronal plane. Anterior means toward the front of the body. Posterior means toward the back of the body. Superior means toward the head. Inferior means toward the feet. Medial means toward the midline of the body. Lateral means away from the midline of the body. Axial means toward a central axis of the body. Abaxial means away from a central axis of the body. Ipsilateral means on the same side of the body. Contralateral means on the opposite side of the body. These descriptive terms may be applied to an animate or inanimate body.

The drill guide 4000 disclosed herein may be included in a system with implants and/or other instruments. For example, the illustrated drill guide 4000 is adapted for glenoid arthroplasty and may be included in a system with the implants and/or instruments disclosed at least in the following copending applications: U.S. Patent Application No. 14/042258, filed on September 30, 2013; U.S. Patent Application No. 15/587895, filed on May 5, 2017; U.S. Patent Application No. 14/592837, filed on January 8, 2015; U.S. Patent Application No. 15/228443, filed on August 4, 2016; and U.S. Patent Application No. 15/653305, filed on July 18, 2017.

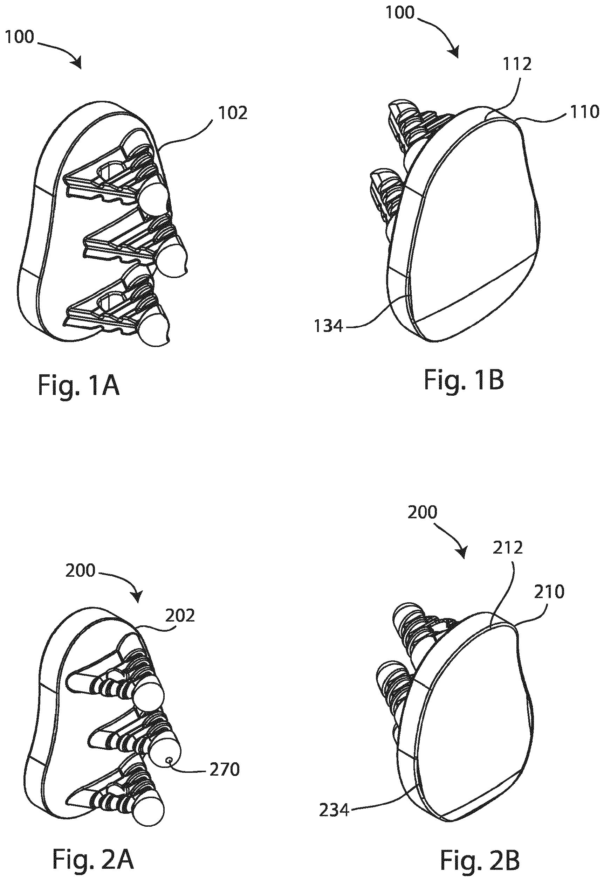

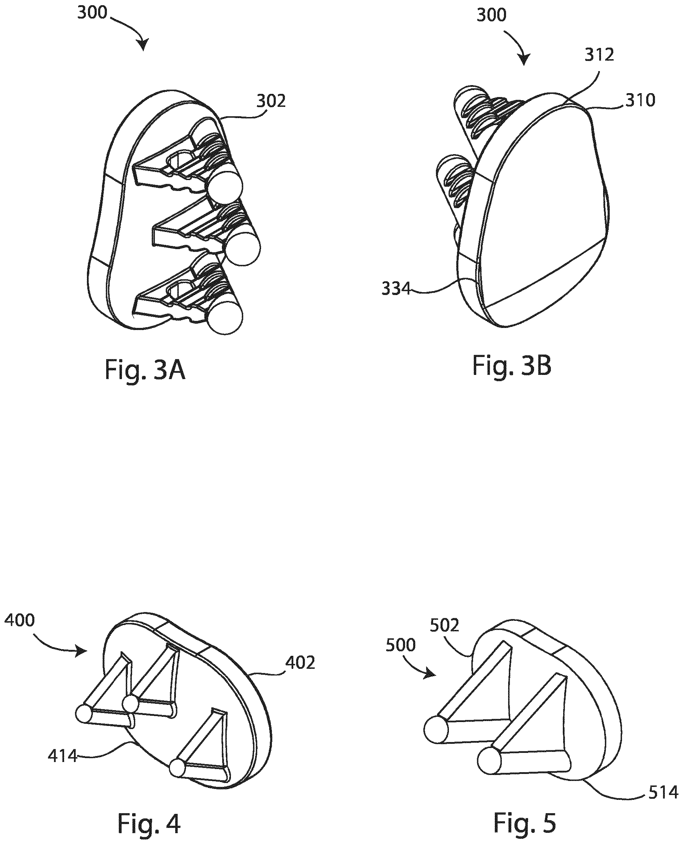

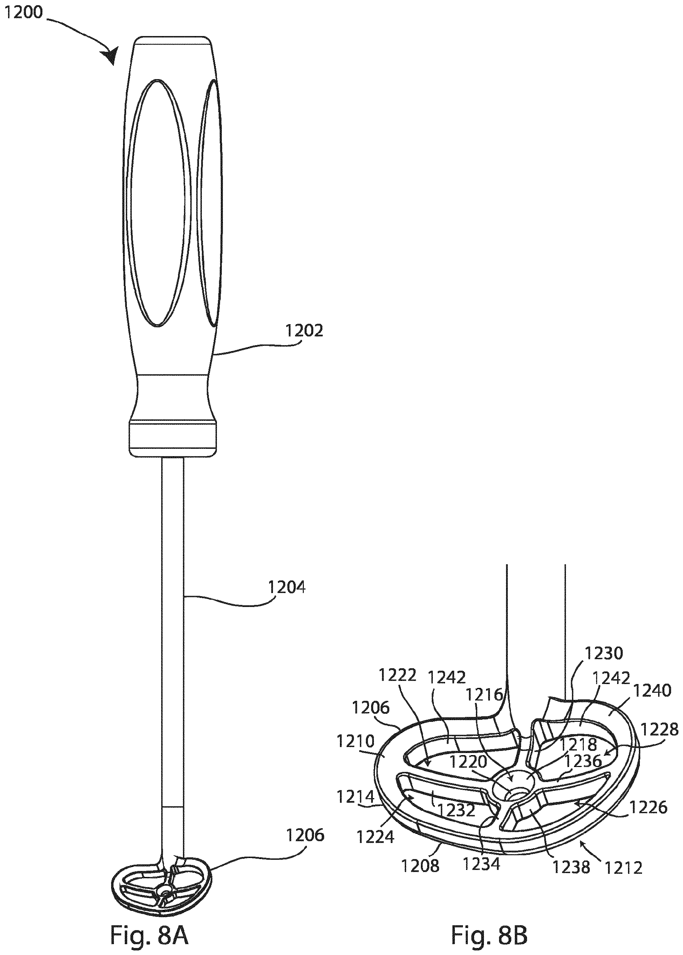

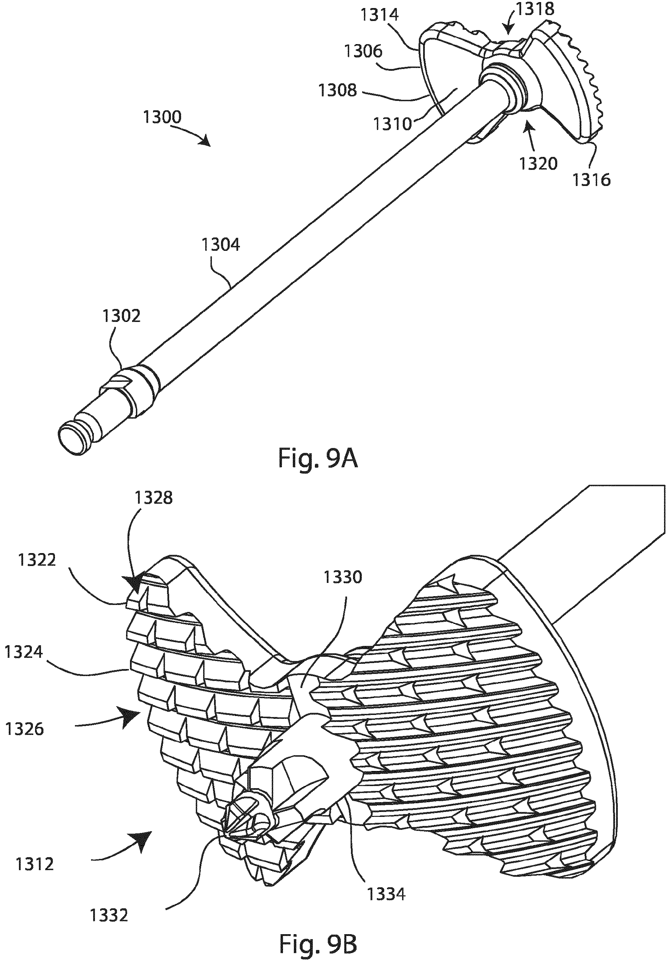

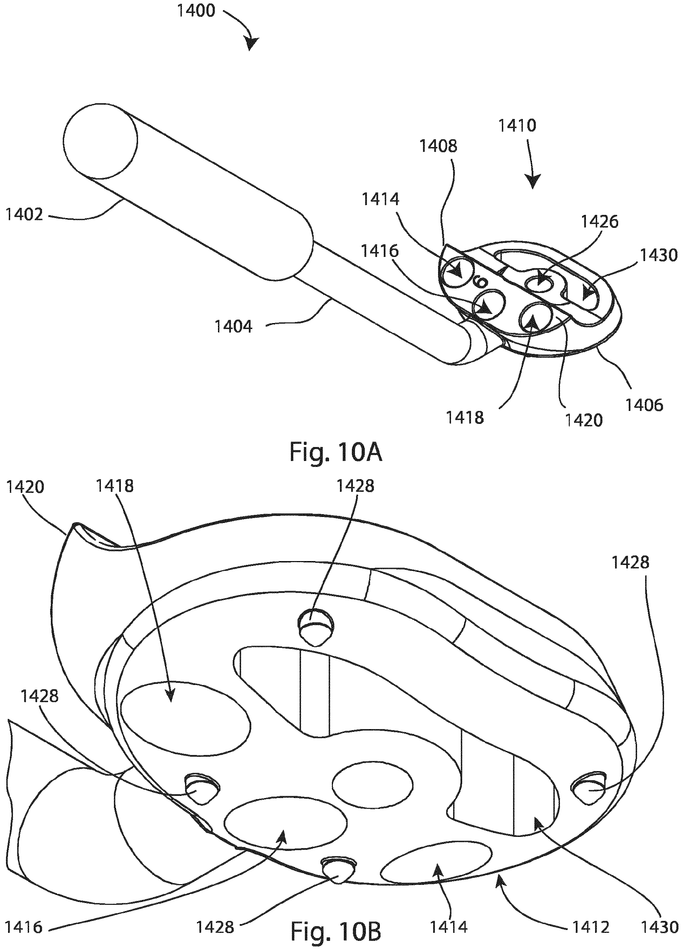

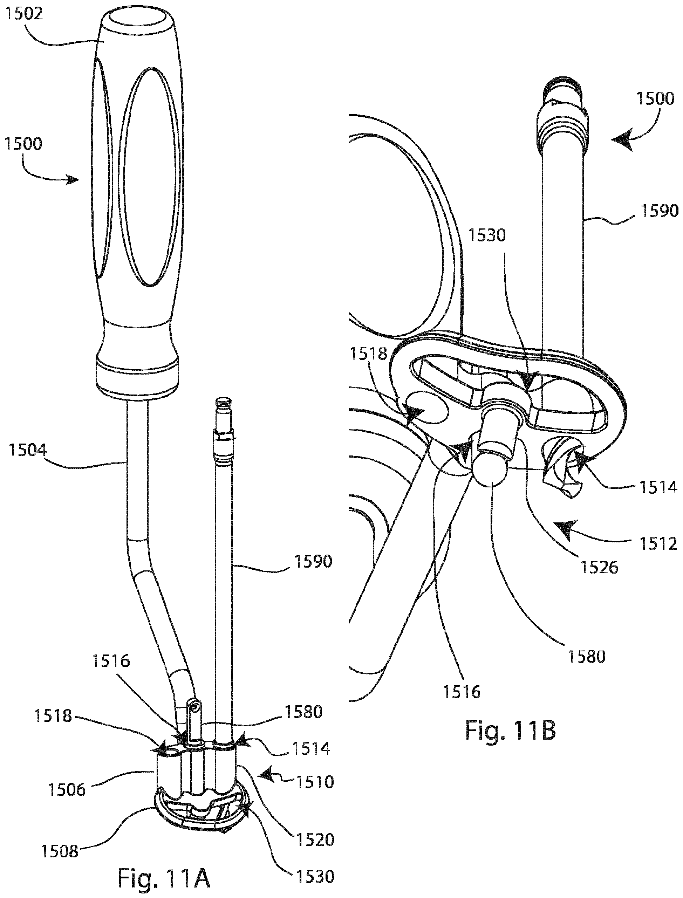

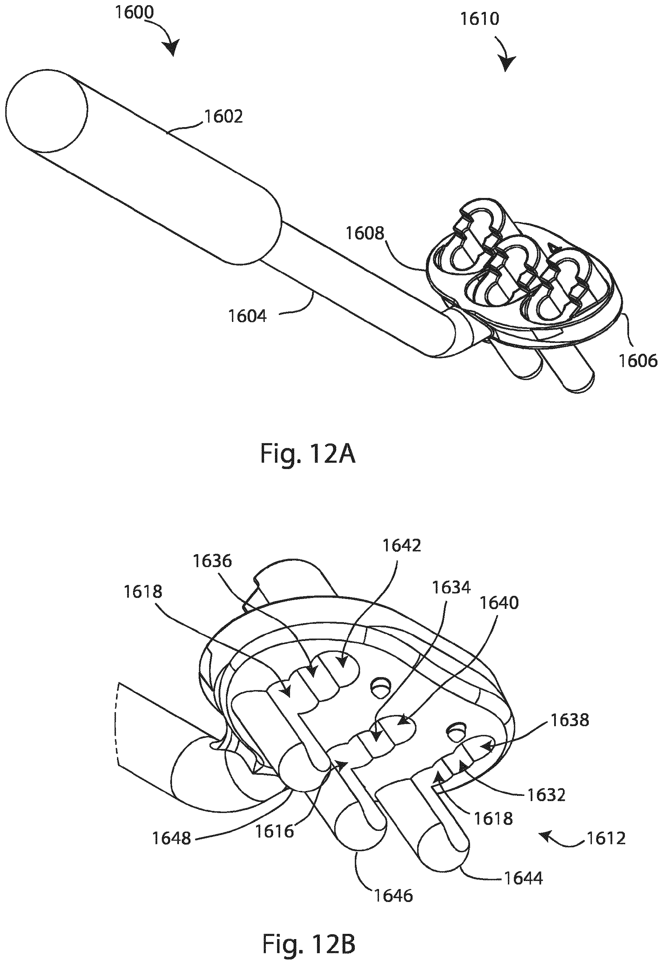

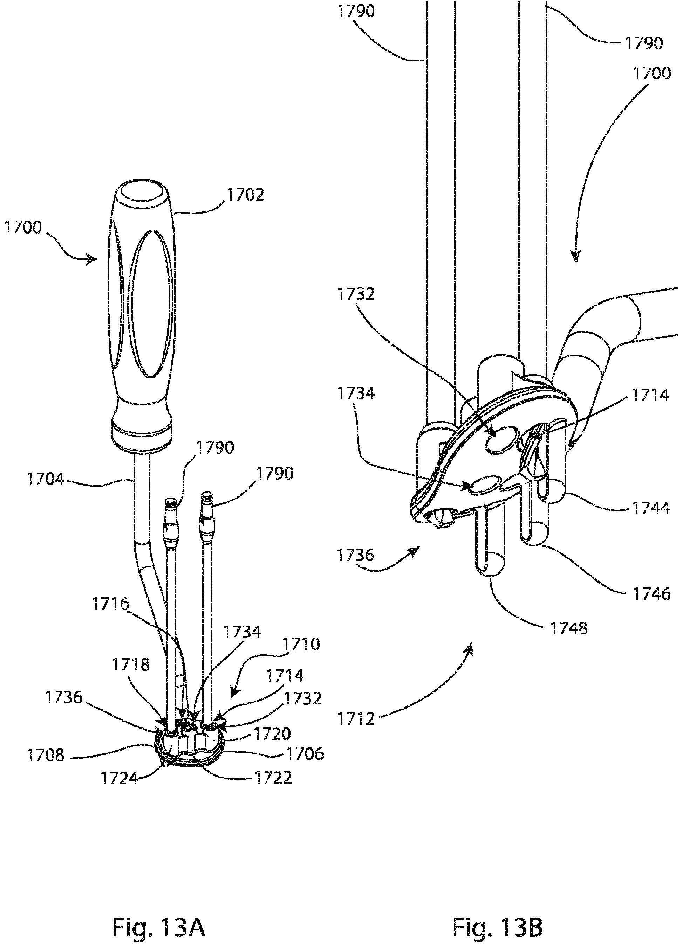

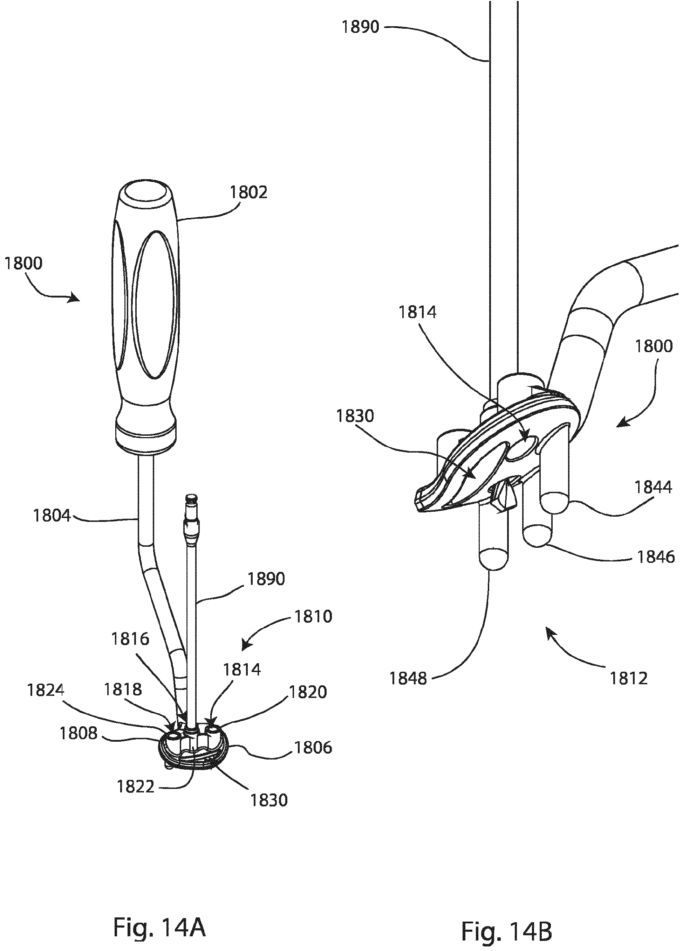

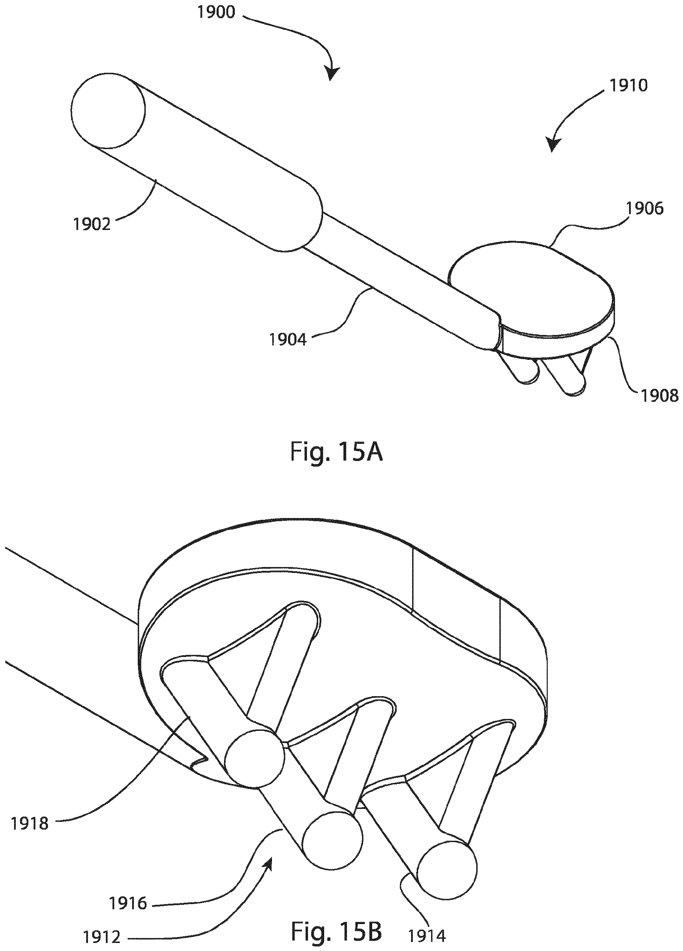

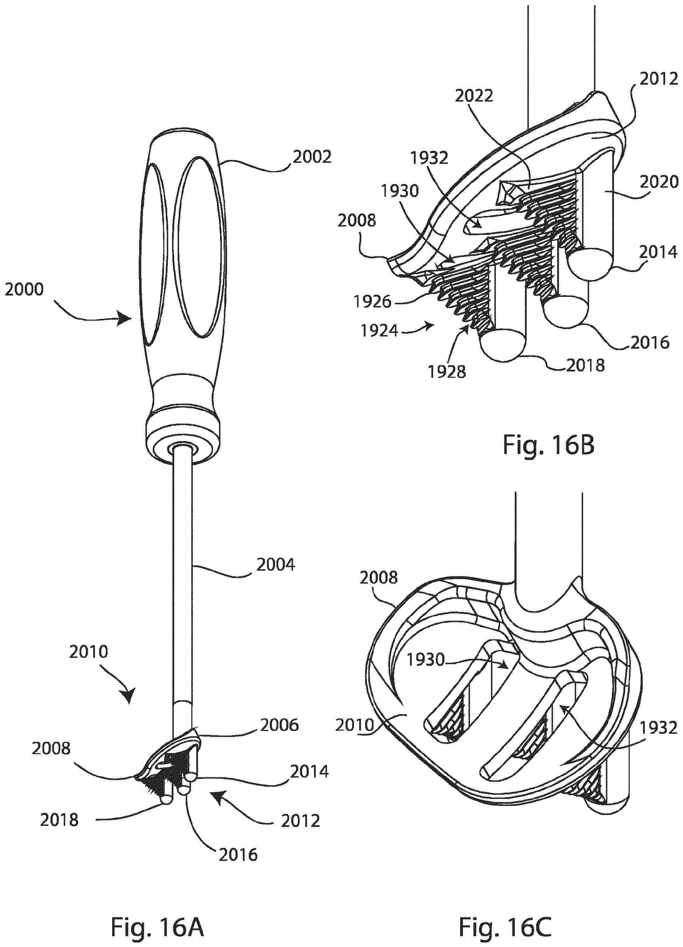

The illustrated drill guide 4000 may be included in a system with the implants and/or instruments disclosed in U.S. Patent Application Nos. 14/042258 and 15/587895, including glenoid components 100, 200, 300, 400, 500, 600, 700 (FIGS. 1A-7B herein); sizing template 1200 (FIGS. 8A-8B herein); reamer 1300 (FIGS. 9A-9B herein); drill guides 1400, 1500, 1600, 1700, 1800 (FIGS. 10A-14B herein); punch 1900 (FIGS. 15A-15B herein); and broach 2000 (FIGS. 16A-16C herein). Furthermore, at least the sizing template 1200 and/or any of the drill guides 1400, 1500, 1600, 1700, 1800 may be adapted according to the principles disclosed herein.

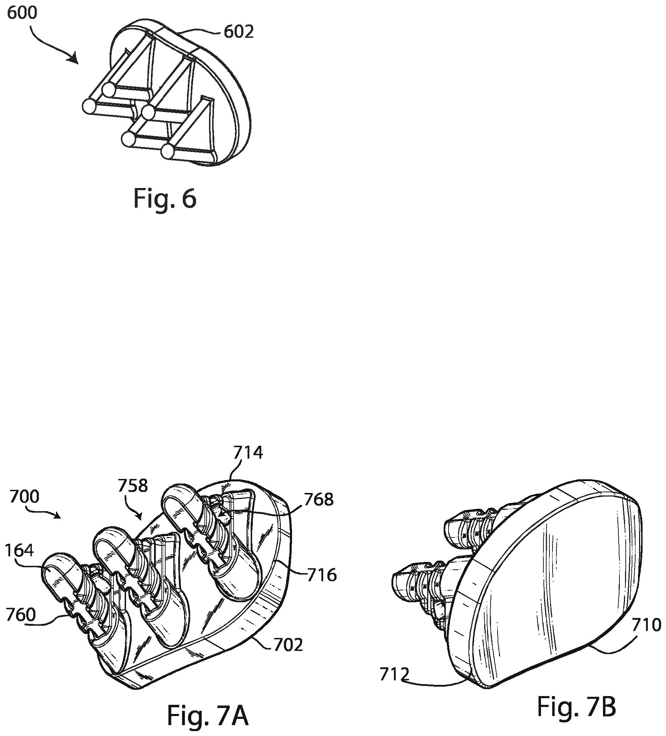

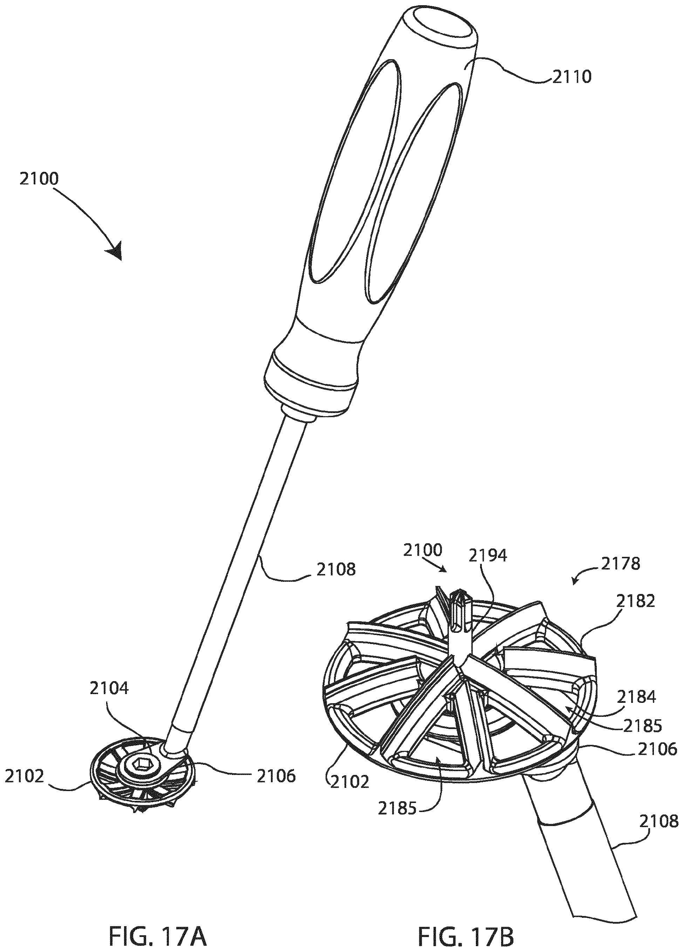

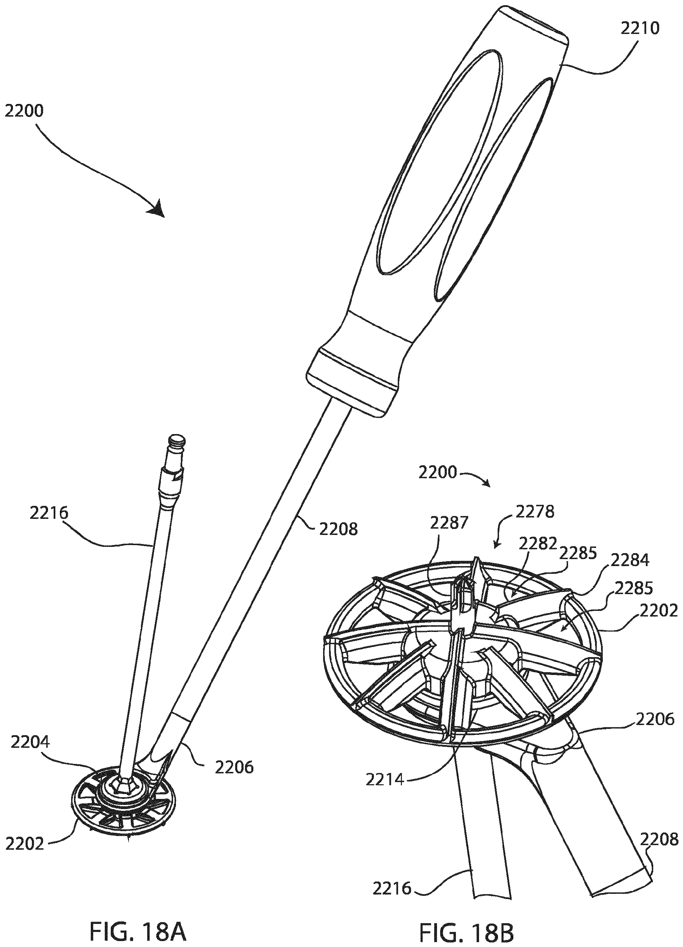

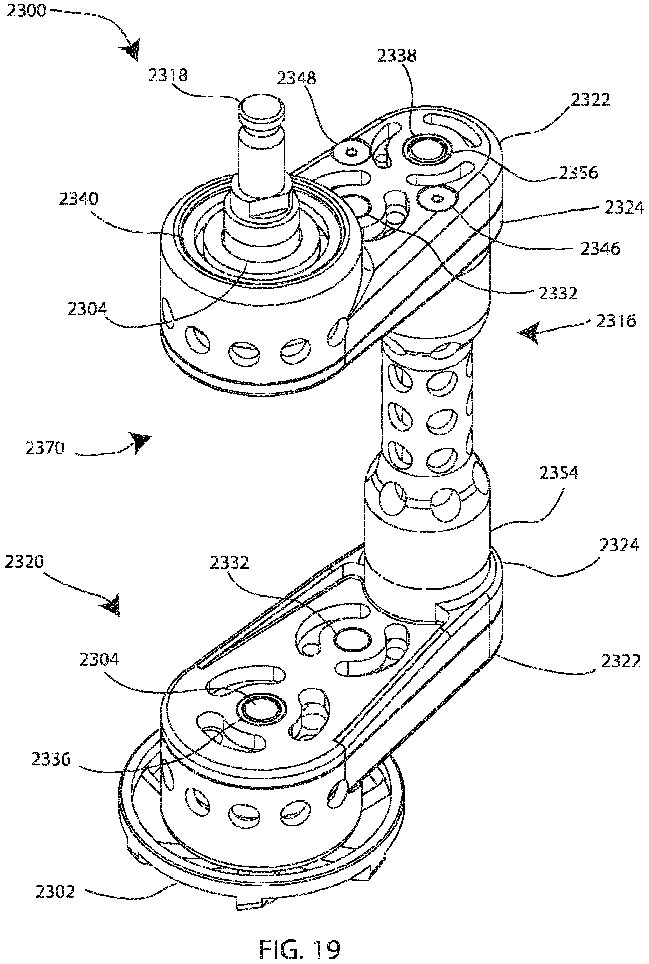

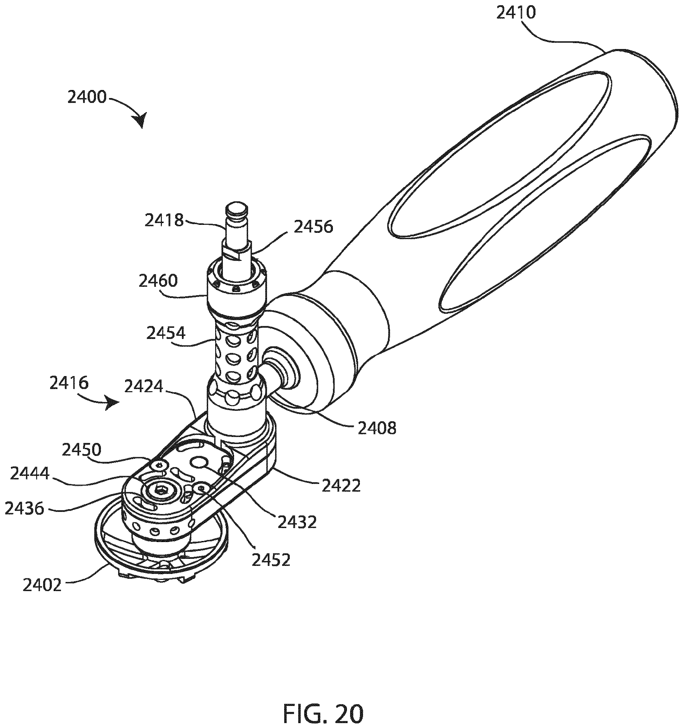

The illustrated drill guide 4000 may be included in a system with the implants and/or instruments disclosed in U.S. Patent Application No. 14/592837, including glenoid component 700 (FIGS. 7A-7B herein), reamer 1300 (FIGS. 9A-9B herein), and offset reamers 2100, 2200, 2300, 2400 (FIGS. 17A-20 herein).

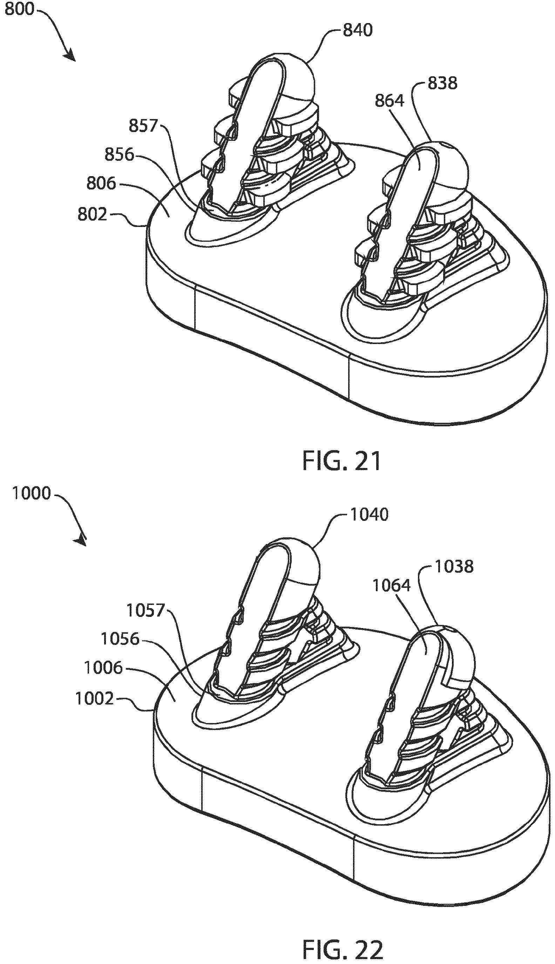

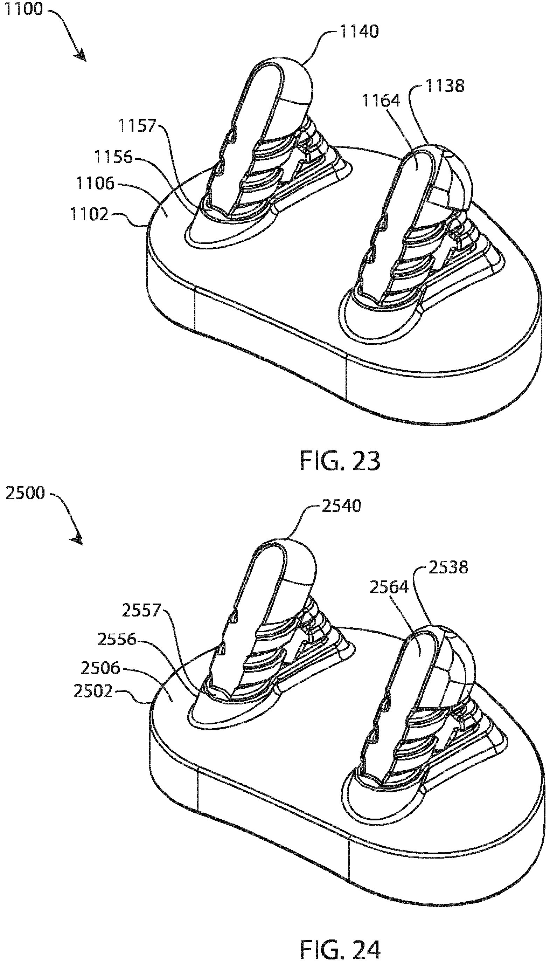

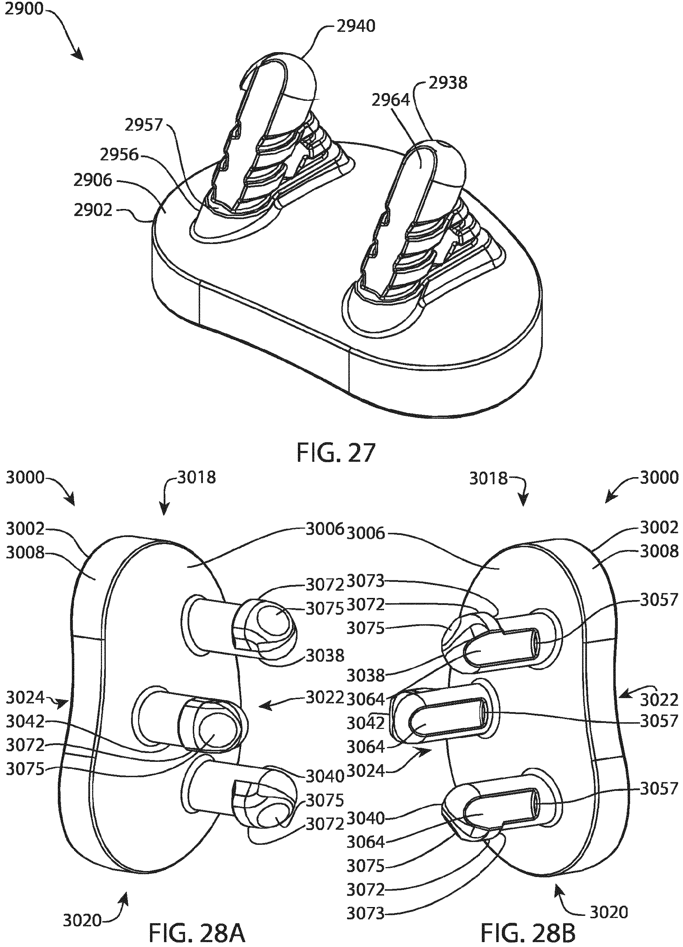

The illustrated drill guide 4000 may be included in a system with the implants and/or instruments disclosed in U.S. Patent Application No. 15/228443, including glenoid components 800, 1000, 1100, 2500, 2700, 2800, 2900 (FIGS. 21-27 herein).

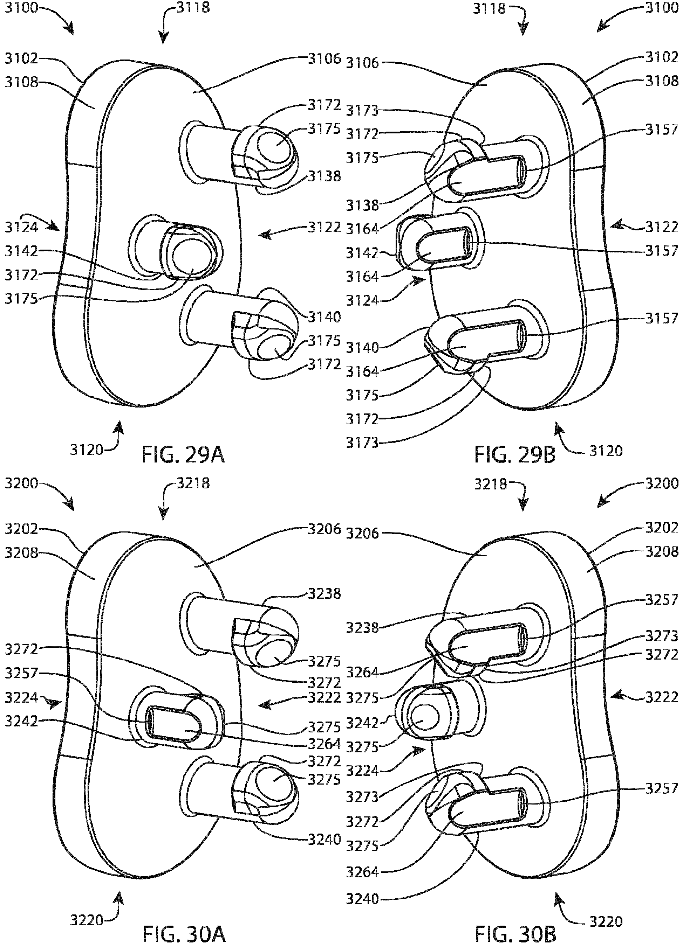

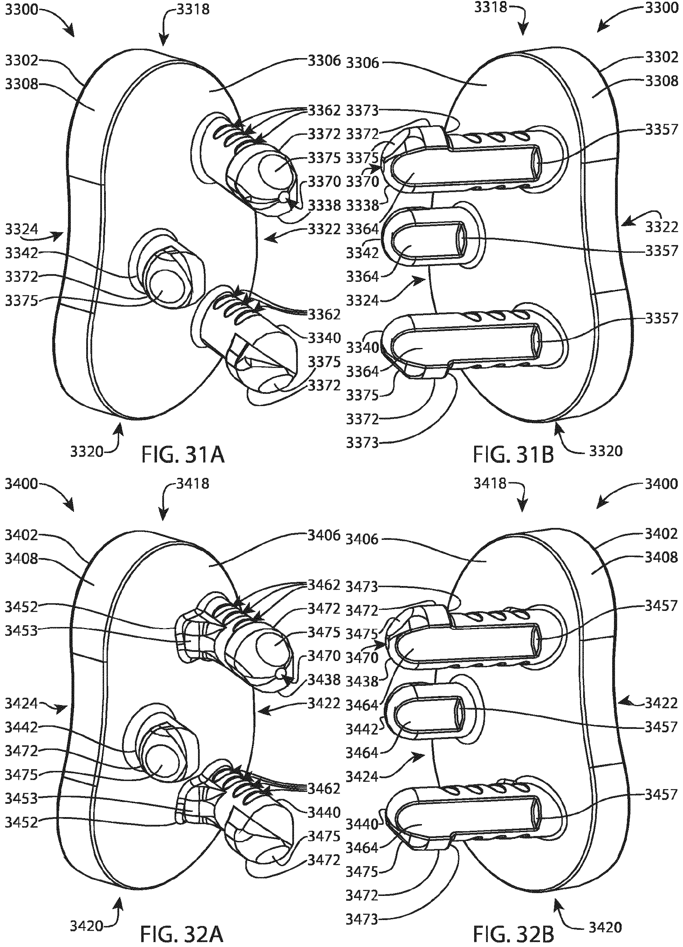

The illustrated drill guide 4000 may be included in a system with the implants and/or instruments disclosed in U.S. Patent Application No. 15/653305, including glenoid components 800, 1000, 1100, 2500, 2700, 2800, 2900, 3000, 3100, 3200, 3300, 3400, 3500, 3600, 3700, 3800, 3900 (FIGS. 21-33F herein).

The drill guide 4000 disclosed herein includes a guide body, or working portion 4200, and a handle 4400 with a shaft 4300. The guide body 4200 includes one or more cylindrical holes 4224, 4226 which serve as the guide(s) for a drill which is actuated through the hole(s). The drill guide 4000 controls the direction and/or the depth to which the drill is advanced into a bone, which dictates the shape of the hole made by the drill in the bone. The handle 4400 is held by the operator, and is used to hold the guide body 4200 against the bone into which the operator is drilling. The handle 4400 is not rigidly fixed to the guide body 4200 as is typical, but instead is coupled to the guide body 4200 by an articulation or joint which allows movement between the two parts. With this design, the operator can apply pressure along the handle 4400 to firmly secure the guide body 4200 against the bone, but once the guide body 4200 is secured against the bone, small movements of the operator's hand and the handle 4400 will not alter the position of the guide body 4200. The articulation or joint permits the handle 4400 to move relative to the guide body 4200, thus the guide body 4200 may remain stationary against the bone while a drill is actuated through the hole(s). This ensures that the holes created by the drill guide 4000 will be accurately placed in the intended position and/or depth and/or trajectory. Without the articulation or joint, small movements of the handle of the drill guide 4000 may alter the trajectory of the drill guide, then the trajectory of the drilled hole, and thus the final resting position of the implant inserted into that hole. Malpositioning of the drill guide, specifically the working portion 4200, may directly correlate to malpositioning of the implant, which puts the implant at risk for loosening or other failure modes.

The articulation may be a hinge joint, a universal joint, a ball-and-socket joint, a polyaxial joint, a saddle joint, or any other joint. A polyaxial joint is a joint that provides for rotation about at least two axes, wherein the second axis intersects or is skew to the first axis. The articulation may be a flexible shaft that couples the working portion 4200 to the handle 4400 or shaft 4300. The articulation may be spring biased. The articulation may be a magnetic joint that includes a magnet acting on a ferrous material, or magnets acting on each other. The articulation may be fixed (so that the guide body 4200 is captive to the handle 4400 or shaft 4300 in use, albeit free to move relative to the handle 4400), or may be dissociated into separate parts (so that the guide body 4200 is removable and connectable to the handle 4400 or shaft 4300 in use). The articulation or joint may include a first joint portion or joint feature carried by the working portion 4200 and a second joint portion or joint feature carried by the handle 4400 or shaft 4300. Some joint designs may include more than just two joint portions or joint features.

In an embodiment, the body 4202 of the drill guide 4000 contains a projection, or spike 4244, on the bone facing side which fits into a corresponding hole on the bone, which serves to resist side-side movement. In another embodiment, the body of the drill guide 4000 contains an additional hole or cannulation on the bone facing side into which a pin or dowel, anchored in the bone, is placed, which also serves to resist side-side movement. These are examples of stabilizing features that stabilize the body 4202 relative to a bone.

Referring to FIG. 34, a drill guide 4000 may include a working portion 4200, a shaft 4300, a pin 4398, and a handle 4400. The working portion 4200 may be coupled to a first end (distal end) of the shaft 4300 by an articulation or a joint, and the handle 4400 may be coupled to a second end (proximal end) of the shaft 4300 opposite the working portion 4200. The shaft 4300 may be free to pivot relative to the working portion 4200 about the articulation or joint, which in this example is a hinge joint formed where the working portion 4200 is coupled to the shaft 4300 by the pin 4398. The handle 4400 may be rigidly fixed to the shaft 4300.

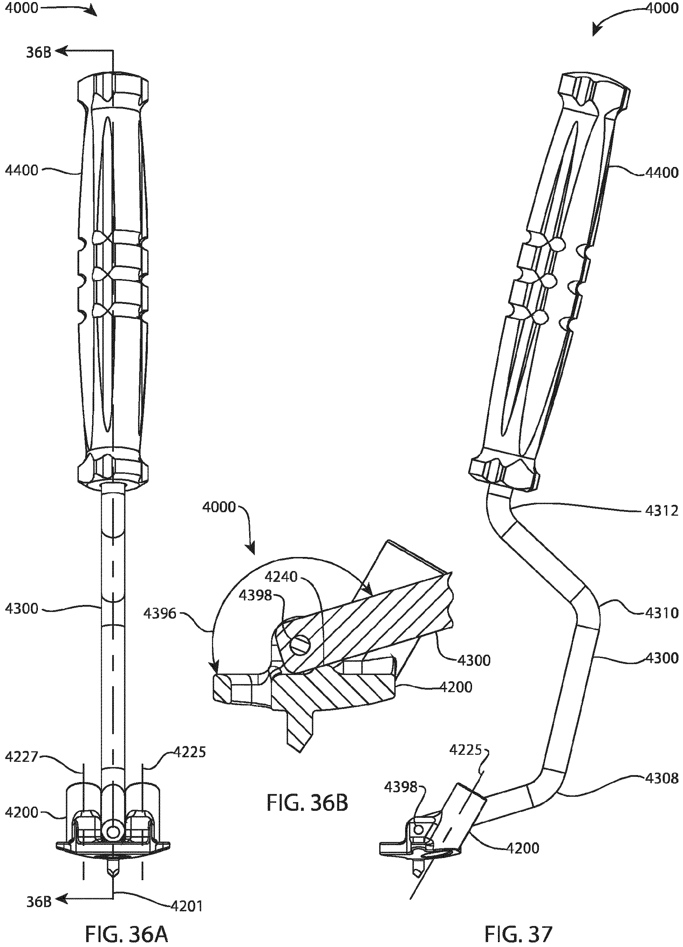

Referring to FIGS. 34-41, the working portion 4200 may be a bilaterally symmetric component with a plane of symmetry 4201 along section line 36B-36B of FIG. 36A.

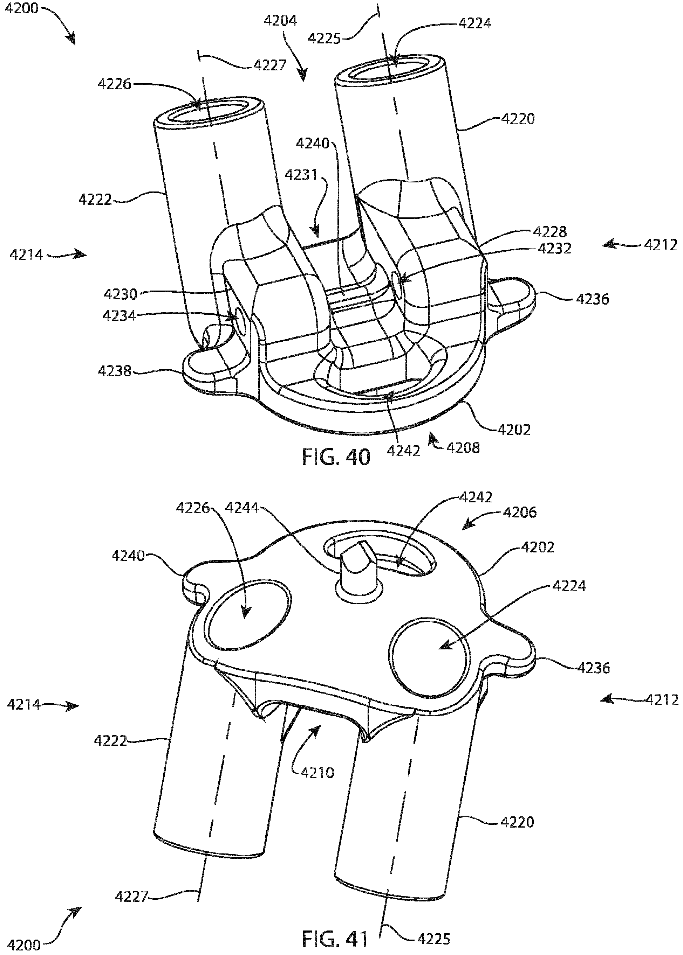

The working portion 4200 may include a body 4202 with an obverse side 4204 and a reverse side 4206 opposite the obverse side. The obverse side 4204 may be referred to as a top side or proximal side, and the reverse side 4206 may be referred to as a bottom side or distal side. The body 4202 may also have a front side 4208, a back side 4210, a left side 4212, and a right side 4214. In use, the reverse side 4206 faces a bone surface such as a glenoid socket, while the obverse side 4204 faces away from the bone surface. The reverse side 4206 may be referred to as a bone facing side, and may make close contact with the bone surface. The reverse side 4206 is shown as a convex spherical surface. However, the reverse side 4206 need not be spherical, and may be flat (planar), concave, or otherwise shaped to complement a particular bone surface. The reverse side 4206 may be used against one or more natural bone surfaces, or against one or more bone resection surfaces, or a combination of natural and resected surfaces. The obverse side 4204 is shown as a planar surface (seen best in FIGS. 36B and 40). However, the obverse side 4204 may be convex, concave, or otherwise shaped as a matter of design choice.

In the example shown, the obverse side 4204 includes a left barrel 4220, a right barrel 4222, a left barrel hole 4224, a right barrel hole 4226, a left barrel hole axis 4225, a right barrel hole axis 4227, a left pin base 4228, a right pin base 4230, a left pin hole 4232, a right pin hole 4234, a left lateral tab 4236, and a right lateral tab 4238. The left and right barrels 4220, 4222 support the left and right barrel holes 4224, 4226, respectively. The left and right barrel holes 4224, 4226 extend completely through the working portion 4200 along the left and right barrel hole axes 4225, 4227, respectively. The left and right barrel hole axes 4225, 4227 are parallel to each other and to the plane of symmetry 4201 in this example, and they run in an oblique direction from obverse-back (top-back) to reverse-front (bottomfront). The left and right pin bases 4228, 4230 support the left and right pin holes 4232, 4234, respectively. The left and right pin holes 4232, 4234 are coaxial and may share the same diameter. Together, the left and right pin holes 4232, 4234 may be thought of as a single pin hole that extends completely through the working portion 4200 from left to right, perpendicular to the plane of symmetry 4201. The left and right pin holes 4232, 4234 may be referred to as a joint portion or joint feature of the working portion 4200; the joint portion or joint feature may also include the left and right pin bases 4228, 4230. The left and right lateral tabs 4236, 4238 project outwardly from the body 4202 and may be located below the left and right pin holes 4232, 4234 or elsewhere. The left and right lateral tabs 4236, 4238 are examples of anatomical reference features that may be included in the drill guide 4000, preferably in the working portion 4200. Along the plane of symmetry 4201, the obverse side 4204 includes a slot 4231, a ridge 4240 and an aperture 4242. The slot 4231 extends from front to back between the left and right pin bases 4228, 4230. The joint portion or joint feature may also include the slot 4231. The ridge 4240 runs from left to right between the left and right pin bases 4228, 4230. The aperture 4242 extends completely through the body 4202 from obverse to reverse near the front side 4208.

The reverse side 4206 includes a central spike 4244. The spike 4244 may include a trocar point as shown, a drill tip, or it may be blunt. The spike 4244 is one example of a stabilizing feature that stabilizes the working portion 4200 relative to a bone. The left and right barrel holes 4224, 4226 and the aperture 4242 extend through the reverse side 4206; the left and right lateral tabs 4236, 4238 extend to the reverse side 4206.

Other embodiments of the working portion may be designed differently from the working portion 4200. For example, other embodiments may be asymmetric instead of bilaterally symmetric, or they may have another type of symmetry besides bilateral symmetry. Other embodiments may lack clearly defined front, back, left, and right sides, although these basic directional terms may still be employed with these embodiments even if some or all of these four sides are indistinguishable from each other. Other embodiments may have only one barrel, barrel hole, barrel hole axis, pin base, pin hole, and/or tab; yet other embodiments may have more than two barrels, barrel holes, barrel hole axes, pin bases, pin holes, and/or tabs. A single barrel hole and its corresponding barrel hole axis may be inclined at an oblique angle relative to the reverse side as shown, or they may be at any other angle relative to the reverse side, including perpendicular or parallel, according to the design constraints for a particular surgical site. Multiple barrel holes and their corresponding barrel hole axes may all be parallel as shown, or they may be arranged at any other relative angle, including oblique, acute, obtuse, or perpendicular, according to the design constraints for a particular surgical site. Multiple barrel holes and their corresponding barrel hole axes may be located as desired on the working portion, for example in a linear array, a circular array, or in another arrangement, according to the need for bone holes for a particular application of the technology. The pin base(s) and pin hole(s) may be replaced with another feature or features to provide any of the types of joints discussed previously. The tab(s) may be oriented according to the anatomical landmarks for a particular surgical site, and may be replaced with grooves or other indicia as a matter of design choice.

Referring to FIGS. 34-39, the shaft 4300 is an elongated part that extends between a first end 4302 (distal end) and an opposite second end 4304 (proximal end). The first end 4302 may be referred to as a working end and the second end 4304 may be referred to as a handle end. The first end 4302 includes a transverse pin hole 4306 which extends completely through the shaft 4300. The hole 4306 may be referred to as a joint portion or joint feature of the shaft 4300. The shaft 4300 may be straight, or it may include one or more bends. Three bends 4308, 4310, 4312 are shown. Of the three, the bend 4308 is closest to the first end 4302, the bend 4312 is closest to the second end 4304, and the bend 4310 is between the bends 4308, 4312. Referring to FIG. 37, the three bends 4308, 4310, 4312 cooperate to bend a first portion of the shaft 4300 between the bend 4312 and the first end 4302 laterally relative to a second portion of the shaft 4300 between the bend 4312 and the second end 4304. Thus, the first portion of the shaft 4300 between the bend 4312 and the first end 4302 is laterally offset relative to the second portion of the shaft 4300. However, in this example, the first end 4302 remains substantially in line with the second portion of the shaft and the second end 4304. The shaft may be designed differently according to the design constraints for a particular surgical site.

The working portion 4200 may be assembled to the first end 4302 of the shaft 4300 by inserting the first end 4302 between the left and right pin bases 4228, 4230 so that the pin hole 4306 is aligned with the left and right pin holes 4232, 4234 and the first portion of the shaft 4300 is oriented as shown in FIG. 37, offset from the back side 4210 of the body 4202. The pin 4398 may then be inserted into the holes 4306, 4232, 4234. The pin 4398 may have a press fit in one of the three holes and a loose fit in the remaining two holes. The shaft 4300 is free to pivot relative to the working portion 4200 about the pin 4398, as indicated by the double ended range of motion arrow 4396 in FIG. 36B. In the example shown, the shaft 4300 has a range of motion 4396 about the pin 4398 between a first position shown in FIG. 36B, in which the shaft 4300 touches the ridge 4240, and a second position, in which the shaft 4300 touches the obverse front portion of the body 4202. The ridge 4240 and the obverse front portion of the body 4202 act as range of motion stops at each end of the range of motion 4396.

The shaft 4300 may be assembled to the handle 4400 by pressing the second end 4304 of the shaft 4300 into a hole 4402 in one end (distal end) of the handle. Alternatively, the second end 4304 and the hole 4402 may be provided with complementary external and internal threads, respectively. Other means for rigid, secure assembly are contemplated. The shaft 4300 and handle 4400 may be detachably coupled together. When the shaft 4300 and the handle 4400 are coupled together, whether permanently or detachably, they may be referred to collectively as a handle portion of the drill guide 4000.

The drill guide 4000 may include an optional biasing element or biasing mechanism (not shown) to bias the shaft 4300 to a particular neutral position relative to the working portion 4200. For example, with reference to FIG. 36B, the shaft 4300 may be biased to the first position. The biasing element or mechanism may be a spring, a group of springs, a magnet, a cam, a drag pin, or the like. This arrangement may be advantageous, particularly when the working portion 4200 is being inserted into a surgical site for use, because it may stabilize the shaft 4300 relative to the working portion 4200 so that the user can control the position of the working portion 4200 against a bone surface. After the working portion 4200 is inserted and positioned against the bone surface, the user may then temporarily overcome the bias to move the shaft 4300 relative to the working portion 4200. The bias may preferably be light enough (low force) so that small unintentional movements of the shaft 4300 and/or handle 4400 are isolated by the joint between the shaft 4300 and the working portion 4200.

The drill guide 4000 may include an optional user-selectable control or lockout (not shown) that stabilizes or immobilizes the shaft 4300 relative to the working portion 4200 when the control is in a first setting and permits free movement of the shaft 4300 relative to the working portion 4200 when the control is in a second setting. For example, a cable or mechanical linkage may be strung through a cannulation in the shaft 4300 to couple a handlemounted control to a mechanism near the joint.

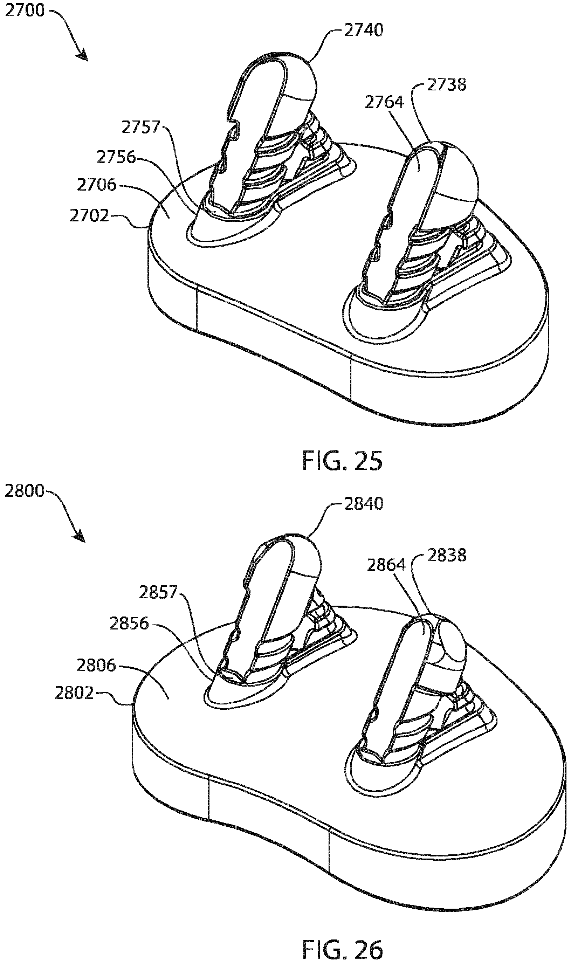

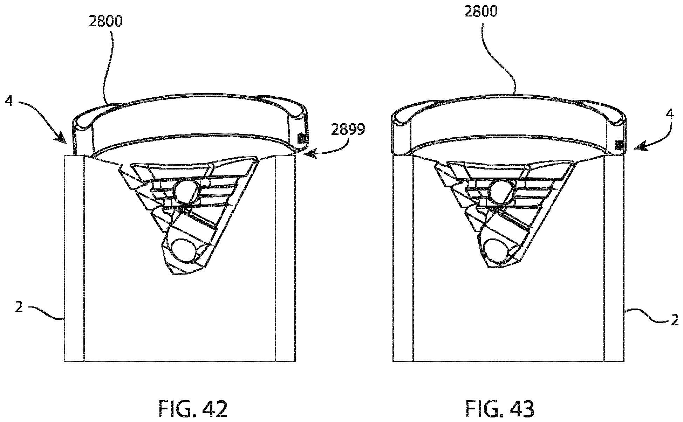

Referring to FIGS. 42 and 43, the glenoid component 2800 of FIG. 26 is shown inserted into prepared bone sockets in the glenoid fossa 4 of a scapula 2. The scapula 2 is shown in a simplified representation. The glenoid component 2800 could be replaced by any of the other glenoid components disclosed herein, along with a complementary bone socket. FIG. 42 illustrates a malpositioned bone socket that is two or three degrees away from proper alignment. Note that the glenoid component 2800 appears to be shifted to the right (anterior) relative to the scapula 2. However, the glenoid component 2800 may actually be tilted, due to its fit in the bone socket, so that the right (anterior) side of the glenoid lifts off of the glenoid and a gap 2899 is present. The right (anterior) side of the glenoid component 2800 may therefore be unsupported, which puts the glenoid component at increased risk of loosening over time, and may cause premature failure of the fixation element due to increased stress. This effect may be exacerbated in a glenoid component designed with a flat bone facing surface and/or a straight fixation peg. Glenoid component failure may cause the patient to undergo revision surgery. Glenoid component loosening is the most common cause of revision shoulder arthroplasty surgery. FIG. 43 illustrates a properly positioned bone socket and glenoid component 2800. This glenoid component is solidly seated on the glenoid and properly supported for long term success.

A method of using the drill guide 4000 does not form part of the invention as claimed and may include some or all of the following steps in any order: inserting the spike 4244 into a complementary hole in a bone surface; positioning the reverse side 4206 against the bone surface; aligning the left and/or right lateral tab 4236, 4238 with one or more anatomical directions or anatomical landmarks; pivoting the shaft 4300 with attached handle 4400 about the pin 4398 relative to the working portion 4200 within the range of motion 4396; and drilling a bone hole through one or both of the left and right barrel holes 4224, 4226.

These steps may be preceded by one or more steps of resecting bone to form one or more resected bone surfaces.

The step of pivoting the shaft 4300 about the pin 4398 relative to the working portion 4200 is advantageous for at least the following reasons.

Firstly, unintentional movements of the user's hand, the handle 4400, and/or the shaft 4300 are not transferred to the working portion 4200 as they would be in a conventional, completely rigid drill guide. More specifically, in the example shown, unintentional movements which cause rotation about the pin 4398 are not transferred. In other examples, the hinge joint formed by the pin 4398 in the holes 4306, 4232, 4234 may be replaced by a ball and socket joint (or other polyaxial joint), in which case unintentional movements which cause rotation about the center point of the ball and socket joint would not be transferred.

Secondly, the user may intentionally pivot the shaft 4300 about the pin 4398 relative to the working portion 4200 so that the shaft 4300 may be used as a retractor or pry bar to lever surrounding anatomical structures out of the way, without disturbing the position of the working portion 4200. In the context of glenoid arthroplasty, for example, the humeral head may be in the way when attempting to drill into the glenoid socket. The shaft 4300 may be pivoted toward the second position to push the humeral head away from its anatomical position relative to the glenoid socket.

Thirdly, the shaft 4300 may be positioned relative to the working portion 4200 to avoid contacting surrounding anatomical structures.

In the context of glenoid arthroplasty, the step of aligning the left and/or right lateral tab 4236, 4238 with an anatomical direction or bony landmark may mean aligning the left and right lateral tabs 4236, 4238 with the superior-inferior direction.

The method may also include the step of inserting a glenoid component into a prepared glenoid fossa of a scapula. This step may include the step of applying bone cement to the prepared glenoid fossa before inserting the glenoid component.

The method may also include steps of using other instruments in conjunction with the drill guide 4000.

Any methods disclosed herein includes one or more steps or actions for performing the described method. The method steps and/or actions may be interchanged with one another. In other words, unless a specific order of steps or actions is required for proper operation of the example, the order and/or use of specific steps and/or actions may be modified.

Reference throughout this specification to "an embodiment" or "the embodiment" means that a particular feature, structure or characteristic described in connection with that embodiment is included in at least one embodiment. Thus, the quoted phrases, or variations thereof, as recited throughout this specification are not necessarily all referring to the same embodiment.

Similarly, it should be appreciated that in the above description of embodiments, various features are sometimes grouped together in a single embodiment, Figure, or description thereof for the purpose of streamlining the disclosure. This method of disclosure, however, is not to be interpreted as reflecting an intention that any claim require more features than those expressly recited in that claim. Rather, as the following claims reflect, inventive aspects lie in a combination of fewer than all features of any single foregoing disclosed embodiment.

Recitation in the claims of the term "first" with respect to a feature or element does not necessarily imply the existence of a second or additional such feature or element.

While specific embodiments and applications of the present technology have been illustrated and described, it is to be understood that the technology is not limited to the precise configuration and components disclosed herein. Various modifications, changes, and variations which will be apparent to those skilled in the art may be made in the arrangement, operation, and details of the systems of the present technology disclosed herein without departing from the scope of the present invention, which is defined by the appended claims.

## Claims

1. A bone drill guide (4000) comprising:
a working portion (4200) comprising a body (4202) having an obverse side (4204) and a bone facing side (4206) opposite the obverse side (4204), a barrel hole (4224, 4226), wherein the barrel hole (4224, 4226) receives a drill with clearance, wherein the bone-facing side (4206) comprises a stabilizing feature that stabilizes the working portion (4200) relative to a bone; and
a handle portion (4400) adapted to be held by the operator;
wherein the handle portion (4400) is coupled to a proximal end (4304) of a shaft (4300) and a distal end (4302) of the shaft (4300) is coupled to the working portion (4200) by a joint which allows movement of the handle (4400) relative to the working portion (4200), so that the operator can apply pressure along the handle (4400) to firmly secure the working portion (4200) against a bone, but once the working portion (4200) is secured against the bone, movement of the handle (4400) will not alter the position of the working portion (4200);
wherein said joint is a hinge joint between the handle portion and the working portion (4200) or a polyaxial joint that provides for rotation about at least two axes, wherein the second axis intersects or is skew to the first axis,
wherein the handle portion (4400) is movable relative to the working portion (4200) about the joint,
wherein the shaft (4300) is free to pivot relative to the working portion (4200) about the joint.

2. The bone drill guide (4000) of claim 1, wherein the joint is a hinge joint between the handle portion and the working portion (4200), optionally wherein the hinge joint is formed where the working portion (4200) is coupled to the distal end of the shaft (4300) by a pin (4398).

3. The bone drill guide (4000) of claim 1, wherein the joint is a polyaxial joint that provides for rotation about at least two axes, wherein the second axis intersects or is skew to the first axis.

4. The bone drill guide (4000) of any preceding claim, wherein the working portion (4200) is captive to the handle portion (4400).

5. The bone drill guide (4000) of any preceding claim, wherein the handle portion (4400) is movable relative to the working portion (4200) about the joint while the drill is actuated in the barrel hole (4224, 4226).

6. The bone drill guide (4000) of any preceding claim, wherein the handle portion (4400) is movable relative to the working portion (4200) about the joint within a range of motion, wherein the working portion (4200) comprises a bone-facing side (4206) and an opposite obverse side (4204), wherein the obverse side (4204) comprises at least one range of motion stop associated with the joint.

7. The bone drill guide (4000) of claim 5, comprising a biasing element that biases the handle portion (4400) to contact the at least one range of motion stop.

8. The bone drill guide (4000) of claim 6, wherein the biasing element is selected from the group consisting of a spring, a magnet, a cam, and a drag pin.

9. The bone drill guide (4000) of claims 5 to 7, wherein the at least one range of motion stop comprises a ridge (4240) on the obverse side (4204), wherein the handle portion (4400) contacts the ridge (4204) at a first end of the range of motion.

10. The bone drill guide (4000) of any of claims 5 to 8, wherein the stabilizing feature comprises a spike (4244) protruding from the bone-facing side (4206).

11. The bone drill guide (4000) of any preceding claim, comprising a locking mechanism comprising a first setting and a second setting;
wherein when the locking mechanism is in the first setting, the handle portion (4400) is immobilized relative to the working portion (4200);
wherein when the locking mechanism is in the second setting, the handle portion (4400) is freely movable relative to the working portion (4200).

12. The bone drill guide (4000) of any preceding claim, wherein the working portion (4200) comprises at least one anatomical reference feature.

13. The bone drill guide (4000) of any preceding claim, wherein the working portion (4200) comprises a pair of outwardly extending anatomical reference tabs (4236, 4238) on opposite sides of the working portion (4200);
optionally wherein when the working portion (4200) is placed against a glenoid socket, the working portion (4200) is positionable so that the pair of outwardly extending anatomical reference tabs (4236, 4238) are aligned along a superior-inferior direction.

14. The bone drill guide (4000) of any preceding claim, wherein the joint is formed by a first joint portion (4232, 4234) and a second joint portion (4306), the working portion (4200) comprising the first joint portion (4232, 4234) and the handle portion (4400) comprising the second joint portion (4306); and/or
wherein the barrel hole (4224, 4226) extends through at least a portion of the working portion (4200).

15. The bone drill guide (4000) of any preceding claim, wherein the shaft (4300) includes one or more bends;
optionally three bends comprising a first bend (4308), a second bend (4310) and a third bend (4312), wherein the three bends (4308, 4310, 4312) cooperate to bend a first portion of the shaft between the third bend (4312) and the distal end (4302) laterally relative to a second portion of the shaft between the third bend (4312) and the proximal end (4304), such that the first portion of the shaft between the third bend (4312) and the distal end (4302) is laterally offset relative to the second portion of the shaft;
further optionally wherein the distal end (4302) remains substantially in line with the second portion of the shaft and the proximal end (4304).

## Patentansprüche

1. Knochenbohrführung (4000), umfassend:
einen Arbeitsabschnitt (4200), der einen Körper (4202) mit einer Vorderseite (4204) und einer dem Knochen zugewandten Seite (4206) gegenüber der Vorderseite (4204), ein Zylinderloch (4224, 4226) umfasst, wobei das Zylinderloch (4224, 4226) einen Bohrer mit Spiel aufnimmt, wobei die dem Knochen zugewandte Seite (4206) ein Stabilisierungsmerkmal umfasst, das den Arbeitsabschnitt (4200) relativ zu einem Knochen stabilisiert; und
einen Griffabschnitt (4400), der angepasst ist, um von dem Bediener gehalten zu werden; wobei
der Griffabschnitt (4400) mit einem proximalen Ende (4304) eines Schafts (4300) gekoppelt ist und ein distales Ende (4302) des Schafts (4300) mit dem Arbeitsabschnitt (4200) durch ein Gelenk gekoppelt ist, das eine Bewegung des Griffs (4400) relativ zu dem Arbeitsabschnitt (4200) ermöglicht, so dass der Bediener entlang des Griffs (4400) Druck ausüben kann, um den Arbeitsabschnitt (4200) fest gegen einen Knochen zu sichern, aber sobald der Arbeitsabschnitt (4200) gegen den Knochen gesichert ist, wird die Bewegung des Griffs (4400) die Position des Arbeitsabschnitts (4200) nicht verändern;
wobei das Gelenk ein Scharniergelenk zwischen dem Griffabschnitt und dem Arbeitsabschnitt (4200) oder ein polyaxiales Gelenk ist, das eine Drehung um mindestens zwei Achsen vorsieht, wobei die zweite Achse die erste Achse schneidet oder schräg zu ihr verläuft,
wobei der Griffabschnitt (4400) relativ zu dem Arbeitsabschnitt (4200) um das Gelenk beweglich ist,
wobei der Schaft (4300) relativ zum Arbeitsabschnitt (4200) frei um das Gelenk schwenken kann.

2. Knochenbohrführung (4000) nach Anspruch 1, wobei das Gelenk ein Scharniergelenk zwischen dem Griffabschnitt und dem Arbeitsabschnitt (4200) ist, optional wobei das Scharniergelenk dort ausgebildet ist, wo der Arbeitsabschnitt (4200) mit dem distalen Ende des Schafts (4300) durch einen Stift (4398) gekoppelt ist.

3. Knochenbohrführung (4000) nach Anspruch 1, wobei das Gelenk ein polyaxiales Gelenk ist, das eine Drehung um mindestens zwei Achsen vorsieht, wobei die zweite Achse die erste Achse schneidet oder schräg zu ihr verläuft.

4. Knochenbohrführung (4000) nach einem der vorhergehenden Ansprüche, wobei der Arbeitsabschnitt (4200) am Griffabschnitt (4400) unverlierbar ist.

5. Knochenbohrführung (4000) nach einem der vorhergehenden Ansprüche, wobei der Griffabschnitt (4400) relativ zu dem Arbeitsabschnitt (4200) um das Gelenk beweglich ist, während der Bohrer in dem Zylinderloch (4224, 4226) betätigt wird.

6. Knochenbohrführung (4000) nach einem der vorhergehenden Ansprüche, wobei der Griffabschnitt (4400) relativ zu dem Arbeitsabschnitt (4200) um das Gelenk innerhalb eines Bewegungsbereichs beweglich ist, wobei der Arbeitsabschnitt (4200) eine dem Knochen zugewandte Seite (4206) und eine gegenüberliegende Vorderseite (4204) umfasst, wobei die Vorderseite (4204) mindestens einen dem Gelenk zugeordneten Bewegungsbereichsanschlag umfasst.

7. Knochenbohrführung (4000) nach Anspruch 5, umfassend ein Vorspannelement, das den Griffabschnitt (4400) vorspannt, um den mindestens einen Bewegungsbereichsanschlag zu berühren.

8. Knochenbohrführung (4000) nach Anspruch 6, wobei das Vorspannelement aus der Gruppe ausgewählt ist, die aus einer Feder, einem Magneten, einem Nocken und einem Schleppstift besteht.

9. Knochenbohrführung (4000) nach Anspruch 5 bis 7, wobei der mindestens eine Bewegungsbereichsanschlag eine Rippe (4240) auf der Vorderseite (4204) umfasst, wobei der Griffabschnitt (4400) die Rippe (4204) an einem ersten Ende des Bewegungsbereichs berührt.

10. Knochenbohrführung (4000) nach einem der Ansprüche 5 bis 8, wobei das Stabilisierungsmerkmal einen Dorn (4244) umfasst, der von der dem Knochen zugewandten Seite (4206) vorsteht.

11. Knochenbohrführung (4000) nach einem der vorhergehenden Ansprüche, umfassend einen Verriegelungsmechanismus, der eine erste Einstellung und eine zweite Einstellung umfasst,
wobei, wenn sich der Verriegelungsmechanismus in der ersten Einstellung befindet, der Griffabschnitt (4400) relativ zu dem Arbeitsabschnitt (4200) immobilisiert ist,
wobei, wenn sich der Verriegelungsmechanismus in der zweiten Einstellung befindet, der Griffabschnitt (4400) relativ zu dem Arbeitsabschnitt (4200) frei beweglich ist.

12. Knochenbohrführung (4000) nach einem der vorhergehenden Ansprüche, wobei der Arbeitsabschnitt (4200) mindestens ein anatomisches Referenzmerkmal umfasst.

13. Knochenbohrführung (4000) nach einem der vorhergehenden Ansprüche, wobei der Arbeitsabschnitt (4200) ein Paar von sich nach außen erstreckenden anatomischen Referenzlaschen (4236, 4238) auf gegenüberliegenden Seiten des Arbeitsabschnitts (4200) umfasst,
optional wobei, wenn der Arbeitsabschnitt (4200) gegen eine Glenoidpfanne platziert ist, der Arbeitsabschnitt (4200) so positionierbar ist, dass das Paar von sich nach außen erstreckenden anatomischen Referenzlaschen (4236, 4238) entlang einer Superior-Inferior-Richtung ausgerichtet ist.

14. Knochenbohrführung (4000) nach einem der vorhergehenden Ansprüche, wobei das Gelenk durch einen ersten Gelenkabschnitt (4232, 4234) und einen zweiten Gelenkabschnitt (4306) gebildet ist, wobei der Arbeitsabschnitt (4200) den ersten Gelenkabschnitt (4232, 4234) umfasst und der Griffabschnitt (4400) den zweiten Gelenkabschnitt (4306) umfasst, und/oder
wobei sich das Zylinderloch (4224, 4226) durch mindestens einen Abschnitt des Arbeitsabschnitts (4200) erstreckt.

15. Knochenbohrführung (4000) nach einem der vorhergehenden Ansprüche, wobei der Schaft (4300) eine oder mehrere Biegungen einschließt;
optional drei Biegungen, die eine erste Biegung (4308), eine zweite Biegung (4310) und eine dritte Biegung (4312) umfassen, wobei die drei Biegungen (4308, 4310, 4312) zusammenwirken, um einen ersten Abschnitt des Schafts zwischen der dritten Biegung (4312) und dem distalen Ende (4302) seitlich relativ zu einem zweiten Abschnitt des Schafts zwischen der dritten Biegung (4312) und dem proximalen Ende (4304) zu biegen, so dass der erste Abschnitt des Schafts zwischen der dritten Biegung (4312) und dem distalen Ende (4302) relativ zum zweiten Abschnitt des Schafts seitlich versetzt ist;
ferner optional, wobei das distale Ende (4302) im Wesentlichen in einer Linie mit dem zweiten Abschnitt des Schafts und dem proximalen Ende (4304) bleibt.

## Revendications

1. Guide de foret à os (4000) comprenant :
une partie de travail (4200) comprenant un corps (4202) possédant un côté avers (4204) et un côté (4206) faisant face à l'os opposé au côté avers (4204), un trou de fourreau (4224, 4226), ledit trou de fourreau (4224, 4226) recevant un foret avec dégagement, ledit côté (4206) faisant face à l'os comprenant un élément de stabilisation qui stabilise la partie de travail (4200) par rapport à un os ; et
une partie poignée (4400) conçue pour être tenue par l'opérateur ;
la partie poignée (4400) étant couplée à une extrémité proximale (4304) d'un arbre (4300) et une extrémité distale (4302) de l'arbre (4300) étant couplée à la partie de travail (4200) par une articulation qui permet le déplacement de la poignée (4400) par rapport à la partie de travail (4200), de sorte que l'opérateur puisse appliquer une pression le long de la poignée (4400) pour fixer fermement la partie de travail (4200) contre un os, mais une fois que la partie de travail (4200) est fixée contre l'os, le déplacement de la poignée (4400) ne modifiera pas la position de la partie de travail (4200) ;
ladite articulation étant une articulation à charnière entre la partie poignée et la partie de travail (4200) ou une articulation polyaxiale qui assure une rotation autour d'au moins deux axes, ledit second axe croisant ou étant en biais par rapport au premier axe,
ladite partie de poignée (4400) étant mobile par rapport à la partie de travail (4200) autour de l'articulation,
ledit arbre (4300) étant libre de pivoter par rapport à la partie de travail (4200) autour de l'articulation.

2. Guide de foret à os (4000) selon la revendication 1, ladite articulation étant une articulation à charnière entre la partie poignée et la partie de travail (4200), éventuellement ladite articulation à charnière étant formée à l'endroit où la partie de travail (4200) est couplée à l'extrémité distale de l'arbre (4300) par une goupille (4398).

3. Guide de foret à os (4000) selon la revendication 1, ladite articulation étant une articulation polyaxiale qui assure une rotation autour d'au moins deux axes, ledit second axe croisant ou étant en biais par rapport au premier axe.

4. Guide de foret à os (4000) selon l'une quelconque des revendications précédentes, ladite partie de travail (4200) étant captive de la partie poignée (4400).

5. Guide de foret à os (4000) selon l'une quelconque des revendications précédentes, ladite partie poignée (4400) étant mobile par rapport à la partie de travail (4200) autour de l'articulation pendant que le foret est actionné dans le trou de fourreau (4224, 4226).

6. Guide de foret à os (4000) selon l'une quelconque des revendications précédentes, ladite partie poignée (4400) étant mobile par rapport à la partie de travail (4200) autour de l'articulation dans une plage de déplacement, ladite partie de travail (4200) comprenant un côté (4206) faisant face à l'os et un côté avers opposé (4204), ledit côté avers (4204) comprenant au moins une butée de plage de déplacement associée à l'articulation.

7. Guide de foret à os (4000) selon la revendication 5, comprenant un élément de sollicitation qui sollicite la partie poignée (4400) pour venir en contact avec ladite au moins une butée de plage de déplacement.

8. Guide de foret à os (4000) selon la revendication 6, ledit élément de sollicitation étant choisi dans le groupe constitué par un ressort, un aimant, une came et une goupille d'entraînement.

9. Guide de foret à os (4000) selon les revendications 5 à 7, ladite au moins une butée de plage de déplacement comprenant une arête (4240) sur le côté avers (4204), ladite partie poignée (4400) entrant en contact avec l'arête (4204) au niveau d'une première extrémité de la plage de déplacement.

10. Guide de foret à os (4000) selon l'une quelconque des revendications 5 à 8, ledit élément de stabilisation comprenant une pointe (4244) dépassant du côté (4206) faisant face à l'os.

11. Guide de foret à os (4000) selon l'une quelconque des revendications précédentes, comprenant un mécanisme de verrouillage comprenant un premier réglage et un second réglage ;
lorsque le mécanisme de verrouillage se trouve dans le premier réglage, ladite partie poignée (4400) étant immobilisée par rapport à la partie de travail (4200) ;
lorsque le mécanisme de verrouillage se trouve dans le second réglage, ladite partie poignée (4400) étant librement mobile par rapport à la partie de travail (4200).

12. Guide de foret à os (4000) selon l'une quelconque des revendications précédentes, ladite partie de travail (4200) comprenant au moins un élément anatomique de référence.

13. Guide de foret à os (4000) selon l'une quelconque des revendications précédentes, ladite partie de travail (4200) comprenant une paire de pattes anatomiques de référence (4236, 4238) s'étendant vers l'extérieur sur des côtés opposés de la partie de travail (4200) ;
éventuellement lorsque la partie de travail (4200) est placée contre une cavité glénoïde, ladite partie de travail (4200) pouvant être positionnée de sorte que la paire de pattes anatomiques de référence (4236, 4238) s'étendant vers l'extérieur soient alignées le long d'une direction hautbas.

14. Guide de foret à os (4000) selon l'une quelconque des revendications précédentes, ladite articulation étant formée par une première partie d'articulation (4232, 4234) et une seconde partie d'articulation (4306), ladite partie de travail (4200) comprenant la première partie d'articulation (4232, 4234) et ladite partie poignée (4400) comprenant la seconde partie d'articulation (4306) ; et/ou
ledit trou de fourreau (4224, 4226) s'étendant à travers au moins une partie de la partie de travail (4200).

15. Guide de foret à os (4000) selon l'une quelconque des revendications précédentes, ledit arbre (4300) comprenant un ou plusieurs coudes ;
éventuellement trois coudes comprenant un premier coude (4308), un deuxième coude (4310) et un troisième coude (4312), lesdits trois coudes (4308, 4310, 4312) coopérant pour plier une première partie de l'arbre entre le troisième coude (4312) et l'extrémité distale (4302) latéralement par rapport à une seconde partie de l'arbre entre le troisième coude (4312) et l'extrémité proximale (4304), de sorte que la première partie de l'arbre entre le troisième coude (4312) et l'extrémité distale (4302) soit décalée latéralement par rapport à la seconde partie de l'arbre ;
en outre éventuellement ladite extrémité distale (4302) restant sensiblement alignée avec la seconde partie de l'arbre et l'extrémité proximale (4304).
